# EUROPEAN PATENT APPLICATION

(11) **EP 3 545 897 A1**
(43) Date of publication of application: **02.10.2019**
(21) Application number: 18164833.8
(22) Date of filing: 29.03.2018
(51) Int. Cl.: A61B 90/98, A61B 5/00

(54) **AN IMPLANTABLE DEVICE**

(71) Applicant: Koninklijke Philips N.V., 5656 AE Eindhoven (NL)
(72) Inventor: WORTELBOER, Pippinus Maarten Robertus, 5656 AE Eindhoven (NL); VAN LIESHOUT, Ron Martinus Laurentius, 5656 AE Eindhoven (NL); VERKRUIJSSE, Willem, 5656 AE Eindhoven (NL); GERHARDT, Lutz Christian, 5656 AE Eindhoven (NL); DELLIMORE, Kiran Hamilton J., 5656 AE Eindhoven (NL)
(74) Representative: de Haan, Poul Erik

(57) **Abstract**

There is provided an implantable device (10). The implantable device (10) comprises a body (12) comprising a part (16) operable to expose a pointed tip (14) at an end of the body (12) upon application of a stimulus. When exposed, the pointed tip (14) is configured to enable the implantable device (10) to be moved through tissue upon application of an external force to the implantable device (10).

## Description

### FIELD OF THE INVENTION

The disclosure relates to an implantable device.

### BACKGROUND OF THE INVENTION

For many types of long-term treatments, health monitoring, and wireless connectivity, there is a growing trend to integrate devices in the body of a subject. These devices are referred to as implantable devices, insertables or subcutaneous implants. The implantable devices are often inserted just below the skin of the subject. In humans, typical examples of implantable devices include medical implantable devices, such as subdermal contraceptive implants (e.g. contraceptive rods) and cardiac monitors. Besides medical implantable devices, non-medical devices are also being inserted in the body of subjects for enhanced sensing (e.g. neodymium magnets for magnetic fields) or for convenience (e.g. radiofrequency identification (RFID) chips for electronic identification). In animals, implantable devices are often used for tracking.

There exist dedicated applicators for the implantation of implantable devices. These dedicated devices are routinely only used after sterilization and anaesthetization of the insertion area. For chip-like implantable devices, a scalpel can be used to make an incision in the skin and the implantable device can be inserted directly in the created pocket. For rod-like implantable devices, a channel is first created by a dedicated device. An example of such a dedicated device is a trocar-in-tube device, which comprises a hollow tube, plunger and rod. In use, the plunger is removed from the hollow tube and the rod is subsequently pushed in place through the tube and finally the tube is retracted.

Once implanted, an implantable device performs its function, which can range from the delivery of drugs to the subject and/or sensing health parameters of the subject at predefined intervals, to providing identification signals for the subject. However, an implantable device has a limited lifetime in that it expires after a certain period of time. For example, the implantable device may be depleted after a certain period of time (e.g. in the case of a drug delivery device), the implantable device may no longer provide reliable measurements (e.g. in the case of a measurement device), encapsulation changes over long periods of time may degrade performance, or the implantable device may no longer be compatible with systems in the environment with which it needs to connect. Thus, an implantable device normally needs to be removed from the body of the subject, either completely or to be replaced by a new implantable device (e.g. at the same location or at a different location).

A clinical setting is recommended for removal of an implantable device due to the risk of possible complications and difficulties associated with the removal. For example, the skin needs to be opened by way of an incision (e.g. using a scalpel) to allow access for the implantable device to be gripped and subsequently pulled out for removal. Moreover, in contrast to the dedicated devices that are available for implantation, only simple surgical tools are currently available for removal. These surgical tools used for removal often result in skin damage and discomfort for the subject. For chip-like implantable devices, the implantable devices can be squeezed out through the incision by hand or pulled out with tweezers or forceps. For rod-like implantable device, the procedure is more delicate as the length-diameter ratio of these implantable devices is around 17 and the diameter of these implantable devices is around 2.5 mm. Moreover, in general, implantable devices are encapsulated and removal can thus require further dissection of the fibrous capsule before it can be pushed to the incision and grasped by tweezers or forceps. The removal of implantable devices also usually involves local anesthetics and manual maneuvering. This requires special skill as the skin around the insert is highly movable and the location at which the incision is to be made can thus displace as soon as a force is exerted.

The force needed to break through the skin is higher than the force needed to cut deeper into tissue below the skin. In particular, the outermost skin layer (namely the stratum corneum) is thin but also hard (or tough) compared to deeper tissue, such as the softer epidermis, dermis and subcutaneous fat and muscle layers. As such, while slowly increasing the incision force, the skin in contact with the cutting tool is mainly pushed away and, at the moment a breaking force is reached, the displacement of the tool briefly accelerates deeper into the tissue (due to a sudden drop in force). In view of this, medical devices exist that are aimed at improving the control over the implantation process by reducing the insertion force and overshoot. For example, piezo-electric transducers have been provided that apply vibratory actuation in lateral, torsional and/or longitudinal direction to pierce the skin. The depth of incision can be measured and forces can be reduced when resistance suddenly drops. However, while these medical devices can help with the implantation process, they are not designed to help with the removal of implantable devices. In particular, the implantation process may require only a puncturing element to create a hole in the skin, whereas the removal process generally requires a cutting device to create an incision by a controlled breaking of the skin at a particular location that enables the target implantable device to be reached.

Another complication is that the implantable device itself can block a puncturing element and disturb the vibratory actuation control. Furthermore, simultaneous palpation and manipulation of the implantable device may become more difficult. The removal of an implantable device can also be difficult as the implantable device can migrate over time and thus the precise location of the implantable device may not be known. There are currently no existing techniques that allow for the relocation of implantable devices to their intended location or to a location appropriate for their removal. Instead, on removal of the implantable device, the use of imaging modalities (e.g. ultra sound or magnetic resonance imaging (MRI)) are required to localize the implantable device and/or a larger than necessary incision (compared to the minimal size of the implantable device) is required to allow for the removal. A larger than necessary incision may be required due to a need to insert grabbing tools for use in removing the implantable device. This increases the risk of infection and the severity of scarring. Another disadvantage is the need for a separate device for the removal of the implantable device and the existing devices to aid in the removal of the implantable device are not as sophisticated as those used for implantation.

### SUMMARY OF THE INVENTION

As noted above, a limitation with existing implantable devices is that the removal of the implantable devices can be difficult (due to, for instance, migration), often requiring the use of a separate device (of a size bigger than the implantable device) to aid with the removal and causing considerable damage to the skin. It would thus be valuable to have an improvement to address the existing problems.

Therefore, according to a first aspect, there is provided an implantable device. The implantable device comprises a body. The body comprises a part operable to expose a pointed tip at an end of the body upon application of a stimulus. When exposed, the pointed tip is configured to enable the implantable device to be moved through tissue upon application of an external force to the implantable device.

In some embodiments, the pointed tip may be housed within the body and the part may be operable to expose the pointed tip by releasing the pointed tip from the end of the body. In some embodiments, the pointed tip may be operable to retract into the end of the body on application of another stimulus.

In some embodiments, the stimulus may comprise any one or more of an acoustic stimulus, a chemical stimulus, a mechanical stimulus, a magnetic stimulus, a thermal stimulus, an electric stimulus, and an electromagnetic radiation stimulus.

In some embodiments, the part operable to expose the pointed tip may comprise a dissolvable cap covering the pointed tip and the stimulus may comprise a chemical that dissolves the cap to expose the pointed tip at the end of the body when the chemical is administered.

In some embodiments, the part operable to expose the pointed tip may comprise a meltable cap covering the pointed tip and the stimulus may induce heat in the cap that melts the cap to expose the pointed tip at the end of the body when the stimulus is applied.

In some embodiments, the part operable to expose the pointed tip may comprise a dissolvable structure holding the pointed tip within the body and the stimulus may comprise a chemical that dissolves the structure to release the pointed tip from the end of the body when the chemical is administered.

In some embodiments, the part operable to expose the pointed tip may comprise a spring held in a compressed state by a mechanism and the stimulus may comprise a disengagement of the mechanism to decompress the spring to release the pointed tip from the end of the body.

In some embodiments, the part operable to expose the pointed tip may comprise a compressible portion and a rigid portion and the stimulus may comprise a force that compresses the compressible portion against the rigid portion to release the pointed tip from the end of the body.

In some embodiments, the part operable to expose the pointed tip may comprise a cap covering the pointed tip and the stimulus may comprise a force that breaks the cap to expose the pointed tip at the end of the body when the force is applied to the cap.

In some embodiments, the part operable to expose the pointed tip may comprise a flexible portion and the stimulus may comprise a force that bends the flexible portion to release the pointed tip from the end of the body when the force is applied to the flexible portion.

In some embodiments, the part operable to expose the pointed tip may comprise an expandable body and the stimulus may comprise a catalyst that causes expansion of the expandable body to release the pointed tip from the end of the body when the expandable body is exposed to the catalyst.

In some embodiments, the part operable to expose the pointed tip may comprise a mechanism tuned to disengage at a predefined frequency and the stimulus may comprise a vibration at the predefined frequency to disengage the mechanism to release the pointed tip from the end of the body when the vibration is applied.

In some embodiments, the part operable to expose the pointed tip and the pointed tip may be threadably engaged and the stimulus may comprise a rotating magnetic field that rotates the pointed tip along the part to release the pointed tip from the end of the body when the rotating magnetic field is applied.

In some embodiments, the part operable to expose the pointed tip may comprise a component configured for activation by a magnetic field, an electromagnetic field, or a radiofrequency signal and the stimulus may comprise the magnetic field, electromagnetic field, or radiofrequency signal that activates the component to release the pointed tip from the end of the body when the magnetic field, electromagnetic field, or radiofrequency signal is applied.

According to the aspects and embodiments described above, the limitations of existing systems are addressed. In particular, according to the above-described aspects and embodiments, the implantable device can be moved through tissue with minimal damage since it is not necessary for an incision to be made to the outside of the skin for the implantable device to be moved. Instead, the implantable device can be moved through tissue without an incision to the external surface of the skin (and optionally also without external tools) due to the exposure of a pointed tip at the end of the body. As the implantable device can be moved through tissue without introducing external tools into the body, the risk of contamination to the body is reduced and scarring due to the movement of the implantable device is avoided. There is therefore provided an improvement that overcomes the existing problems.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Figs. 1-10 are illustrations of implantable devices according to embodiments;
Figs. 11-15 are illustrations of removal and/or manipulation devices for use with an implantable device according to embodiments;
Fig. 16 is an illustration of an implantable device according to an embodiment;
Fig. 17 is an illustration of a removal and/or manipulation device for use with an implantable device according to an embodiment; and
Fig. 18 is an illustration of an implantable device according to an embodiment.

### DETAILED DESCRIPTION OF EMBODIMENTS

As noted above, there is provided herein an improvement that overcomes existing problems. More specifically, there is provided herein an implantable (or insertable, or subcutaneous) device that overcomes existing problems. The implantable device described herein can be any device suitable for implantation under the skin or in the tissue (e.g. in the dermis, organs, or any other tissue) of a subject. The subject can be any type of subject, such as a human (e.g. a patient) or an animal. The implantable device can be configured for any purpose. For example, the implantable device can be a medical implantable device according to some embodiments or a non-medical implantable device according to other embodiments.

Examples of a medical implantable device include, but are not limited to, an implantable device configured to provide a contraceptive to the subject, an implantable device configured to deliver a drug to the subject, an implantable device configured to monitor physiological characteristics of the subject (such as the heart rate characteristics of the subject, or any other physiological characteristics, or any combination of physiological characteristics, of the subject), or any other type of medical implantable device, or any combination of medical implantable devices. Examples of a non-medical implantable device include, but are not limited to, an implantable device configured to identify the subject (such as a radiofrequency identification (RFID) chip for electronic identification of the subject), an implantable device configured to track the subject, or any other type of non-medical implantable device, or any combination of non-medical implantable devices. Although some examples have been provided for the type of implantable device in terms of its purpose, it will be understood that any other type of implantable device and any combination of types of implantable devices are possible.

Briefly, the implantable device according to this disclosure comprises a body. In some embodiments, the body may be an elongate body. For example, the body may comprise a cylindrical body. In other embodiments, the body may be transformable (e.g. shapeable) into an elongate body. For example, the body may be a spherical body that can be transformed into an elongate body by extending the spherical body in one direction and/or shrinking the spherical body in another direction. The body comprises a part operable to expose a pointed tip at an end of the body upon application of a stimulus. When exposed, the pointed tip is configured to enable the implantable device to be moved through tissue upon application of an external force to the implantable device. Thus, in effect, the applied stimulus can act as a trigger to activate the pointed tip. In some embodiments, the pointed tip may be activated only upon application of the stimulus. According to some embodiments, during normal operation of the implantable device, the pointed tip may not be in contact with (or may not be in direct contact with) the tissue. The stimulus may be applied to cause exposure of the pointed tip at the end of the body when the implantable device is to be moved through tissue. For example, the stimulus may be applied (e.g. immediately) prior to application of the external force to move the implantable device through tissue.

The pointed tip can be any tip that is pointed to enable the implantable device to be moved through tissue. The pointed tip can, for example, be any shape provided that its shape enables the implantable device to be moved through tissue. In some embodiments, the pointed tip can be sufficiently sharp to enable the implantable device to be moved through tissue. In some embodiments, the pointed tip can be an integral part of the body. In other embodiments, the pointed tip may be directly or indirectly connected (or joined) to the body. Similarly, in some embodiments, the part operable to expose the pointed tip can be an integral part of the body and, in other embodiments, the part operable to expose the pointed tip can be directly or indirectly connected (or joined) to the body. In some embodiments, the body may have a cylindrical shape. However, other shaped bodies are also possible.

The stimulus, upon application of which the part is operable to expose the pointed tip, can comprises any one or more of an acoustic stimulus (e.g. ultrasound), a chemical stimulus (e.g. a photo-chemical stimulus, a local anesthesia, or any other chemical stimulus, or any combination thereof), a mechanical stimulus (e.g. a photo-mechanical stimulus, or any other mechanical stimulus, or any combination thereof), a magnetic stimulus (e.g. a magnetic field, or any other magnetic stimulus, or any combination thereof), a thermal stimulus, an electric stimulus (e.g. an electroactive polymer (EAP), a radiofrequency (RF) wake up signal, or any other electric or electrical stimulus, or any combination thereof), an electromagnetic radiation stimulus, or any other stimulus, or any combination of stimuli, suitable for causing the part to expose the pointed tip. In some embodiments, the stimulus may be applied directly to the part operable to expose the pointed tip. For example, in some embodiments involving a chemical and/or mechanical stimulus, the chemical and/or mechanical stimulus may be applied directly by injecting a chemical into the part operable to expose the pointed tip and/or by mechanically operating the part operable to expose the pointed tip. In other embodiments, the stimulus may be applied indirectly to the part operable to expose the pointed tip. For example, in some embodiments, the stimulus may be applied to the part operable to expose the pointed tip via a cascade of triggers. In some embodiments involving a chemical and/or mechanical stimulus, the chemical and/or mechanical stimulus may be applied indirectly by inducing photons (e.g. using a laser pulse) in the part operable to expose the pointed tip.

The external force can be any force external to the skin that is suitable for moving the implantable device through tissue. Examples of an external force that may be applied to the implantable device to move the implantable device through tissue included, but are not limited to, an external manual force (e.g. an interaction of a finger of a person with the implantable device from outside the skin), an external mechanical force (e.g. an interaction of an external removal device, such as tweezers or forceps, with the implantable device from outside the skin), an external magnetic force, an external electromagnetic force, or any other external force, or any combination of external forces, suitable for moving the implantable device through tissue. In some embodiments, the external force may comprise any one or more of an external pushing force, an external pulling (or grasping) force, an external squeezing force, or any other external force that is capable of moving the implantable device through tissue.

In some embodiments, the pointed tip can be configured to enable the implantable device to be moved through tissue by displacing, separating, parting and/or cutting the tissue upon application of the external force to the implantable device. Thus, for example, the pointed tip can be configured to induce a volume change in the tissue to enable the implantable device to be moved through tissue. In effect, the pointed tip can be configured to pave the way for and/or steer the implantable device to move through tissue. In some embodiments, the pointed tip can be configured to enable the implantable device to be moved through tissue to different locations in the tissue (e.g. in the dermis) upon application of the external force to the implantable device. In this way, where the implantable device migrates or drifts to a location away from an intended (e.g. optimal or ideal) location, the implantable device can be moved back to the intended location. Similarly, where the implantable device needs to be used at various locations within the body, the implantable device can be moved to these various locations. The movement of the implantable device can be achieved without the need to make an incision into the skin and into the underlying tissue, which ensures minimal damage to the skin and underlying tissue. In this way, reposition of the implantable device in the skin can be performed regularly, e.g. during check-ups (such as by using the hands or a tool), in an easy and non-invasive manner.

In some embodiments, the pointed tip can be configured to enable the implantable device to only be moved through tissue upon application of the external force to the implantable device. In other embodiments, the pointed tip can further be configured to enable the implantable device to be moved through tissue to puncture the stratum corneum of the skin upon application of the external force to the implantable device. The pointed tip can be configured to enable the implantable device to puncture the stratum corneum of the skin to allow removal of the implantable device from the skin upon application of the external force to the implantable device. In this way, the implantable device can be removed from the skin without the need for an incision to be made to the outside of the skin. Thus, the risk of contamination to the body is reduced.

Moreover, while removal devices may be used to aid in the removal of the implantable device from the skin, the implantable device can be removed from the skin without such removal devices. In other embodiments, removal devices may be used but in a minimally invasive manner since at least the pointed tip moves through the hole caused by the pointed tip puncturing the skin from the inside out and thus at least the pointed tip can be grasped with a removal device. The hole caused by the pointed tip is a minimal sized skin puncture. In this way, the implantable device described herein limits or even prevents damage to the skin. Moreover, the rapid displacement (i.e. the overshoot) of an incision tool that occurs when breaking the stratum corneum from the outside, which can damage the tissue, is prevented since the implantable device can break the stratum corneum itself from the inside. The pointed tip creates only a minimal path (or expulsion channel) through which the implantable device can move. Also, by having a pointed tip for moving the implantable device inside the body, the pointed tip can be maneuvered just below the stratum corneum and this can provide a target location (which can be more or less fixed) that is visible from the external surface of the skin. This can be useful for locating the implantable device in the skin and/or removing the implantable device from the skin.

In some embodiments, the body of the implantable device may comprise the pointed tip. For example, the pointed tip may be housed within (or positioned inside or embedded inside) the body of the implantable device. In these embodiments, the part operable to expose a pointed tip can be operable to expose the pointed tip by releasing the pointed tip from the end of the body. In this way, the pointed tip can be stored safely inside the implantable device. In some of these embodiments, the pointed tip may be operable to retract into the end of the body on removal of the applied stimulus (or on reduction of the applied stimulus) or on application of another stimulus. Thus, in some embodiments, the part operable to expose the pointed tip may be operable to temporarily expose the pointed tip at the end of the body upon application of the stimulus. In effect, in some embodiments, the pointed tip can be switched between being exposed and being unexposed (e.g. covered). The exposure of the pointed tip at the end of the body is thus reversible according to some embodiments. This can, for example, be useful following the manipulation of an implantable device to move the implantation device to a different location since the pointed tip can be exposed during the manipulation and unexposed following the manipulation to minimize migration.

In some embodiments, the stimulus applied to retract the pointed tip into the end of the body may be the same type of stimulus as the stimulus applied to expose the pointed tip at the end of the body. For example, where the stimulus applied to expose the pointed tip at the end of the body is a mechanical stimulus, the stimulus applied to retract the pointed tip into the end of the body may also be a mechanical stimulus. In other embodiments, the stimulus applied to retract the pointed tip into the end of the body may be a different type of stimulus to the stimulus applied to expose the pointed tip at the end of the body. For example, where the stimulus applied to expose the pointed tip at the end of the body is a chemical stimulus, the stimulus applied to retract the pointed tip into the end of the body may be a mechanical stimulus.

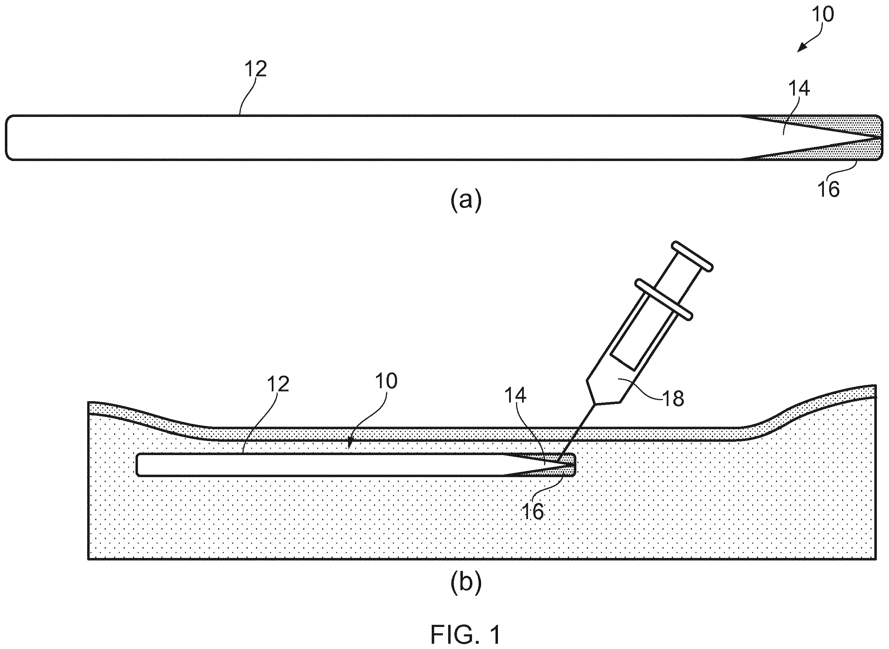

Fig. 1(a) illustrates an example implantable device 10 according to an embodiment and Fig. 1(b) illustrates the example implantable device 10 in use according to an embodiment. As illustrated in Fig. 1(a) and Fig. 1(b), the implantable device 10 comprises a body 12. As described earlier, the body 12 comprises a part 16 operable to expose a pointed tip 14 at an end of the body 12 upon application of a stimulus 18, as illustrated in Fig. 1(b). As also described earlier, when exposed, the pointed tip 14 is configured to enable the implantable device 10 to be moved through tissue upon application of an external force (not illustrated) to the implantable device 12.

In the illustrated example embodiment of Fig. 1(a) and Fig. 1(b), the part 16 operable to expose the pointed tip 14 comprises a dissolvable cap (or retainer) 16 covering the pointed tip 14. The pointed tip 14 can thus be protected by the cap 16. The stimulus 18 according to the illustrated example embodiment of Fig. 1(a) and Fig. 1(b) comprises a chemical (or agent) that dissolves the cap 16 to expose the pointed tip 14 at the end of the body 12 when the chemical 18 is administered. Thus, according to this illustrated example embodiment, the pointed tip 14 can be exposed after dissolution of the cap 16 by the administered chemical.

The cap 16 can be made of any material suitable of being dissolved by a chemical (or agent). In some embodiments, the cap 16 can be formed of a material that is capable of maintaining its structure in the tissue until the cap 16 comes into contact with the chemical that is administered to dissolve the cap 16. The cap 16 can be made of a material that is safe to use in the body, such as a biocompatible material. An example of a material that can be used for the cap 16 is cellulose or any of its derivatives (e.g. from the cellulose ester family), such as cellulose acetate propionate (CAP) or a cellulose acetate (CA). In some embodiments, the material of the cap 16 may be any material that can survive being in contact with the tissue but that is dissolvable by the chemical. In some embodiments, the material of the cap 16 may be a material that can be absorbed by the body once dissolved.

The chemical 18 that is used to dissolve the cap 16 can be administered in any suitable way. For example, the chemical 18 can be administered to the tissue (e.g. surrounding the cap 16) or to the cap 16 itself (e.g. through the tissue). As illustrated in Fig. 1(b), in some embodiments, the chemical 28 can be administered by injecting the chemical 28 through the tissue. For example, in some embodiments, the chemical 18 can be injected through the tissue using a syringe. The combination of the injection of the chemical and the removal of the implantable device from the inside out is less severe than cutting the skin from the outside. In other embodiments (not illustrated), the chemical 18 can be applied to the external surface of the skin. For example, in some embodiments, the chemical 18 can be in the form of a topical cream comprising particles that can penetrate the skin. In some embodiments, the particles that can penetrate the skin may also be attracted to the pointed tip 14. The particles can then release small amounts of dissolvent according to some embodiments.

The chemical 18 may be any chemical suitable to dissolve the cap 16. The chemical 18 can be one that is safe to use in the body, such as a biocompatible chemical and/or a chemical that can dissolve in the body (e.g. a chemical that can dissolve in water). Examples for the chemical 18 include, but are not limited to, polyvinyl alcohol, an ionic liquid anesthetic (e.g. which dissolves cellulose), such as lidocaine-ibuprofen ionic liquid, dimethyl sulfoxide (DMSO). In some embodiments, the chemical 18 may be administered together with anesthetics. In some embodiments, photodegradation may be used to dissolve the cap 16. In some embodiments, the cap 16 may only be partially dissolved by the chemical 18. For example, a partial breakdown of the cap 16 may occur upon application of the chemical 18. In some of these embodiments, pre-stress may aid in breaking the cap 16.

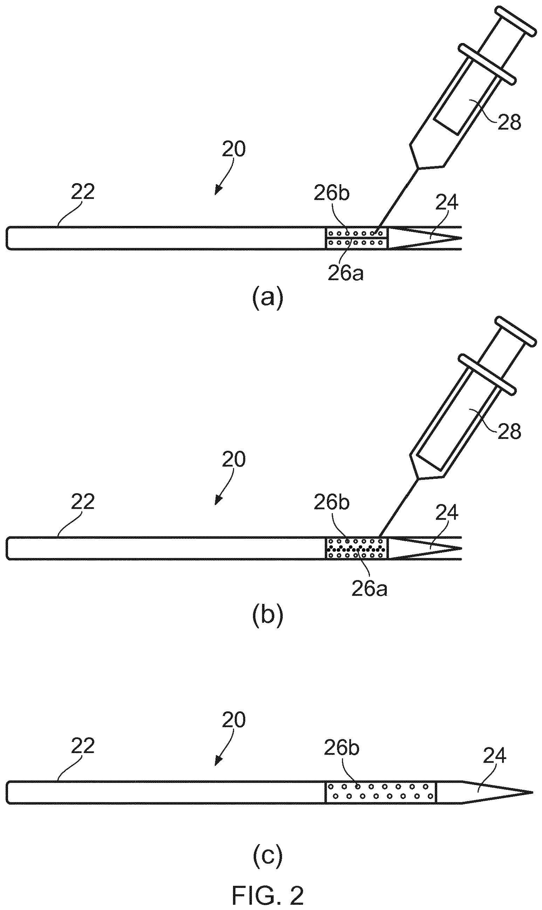

Fig. 2 illustrates an example implantable device 20 according to another embodiment. As illustrated in Fig. 2, the implantable device 20 comprises a body 22. As described earlier, the body 22 comprises a part 26a, 26b operable to expose a pointed tip 24 at an end of the body 22 upon application of a stimulus 28. In the illustrated example embodiment of Fig. 2, the pointed tip 24 can be stored inside the implantable device 20 (e.g. in a retracted state) prior to application of the stimulus 28. As also described earlier, when exposed, the pointed tip 24 is configured to enable the implantable device 20 to be moved through tissue upon application of an external force (not illustrated) to the implantable device 20.

In the illustrated example embodiment of Fig. 2, the part 26a, 26b operable to expose the pointed tip 24 comprises a dissolvable structure (or mechanism) 26a holding the pointed tip 24 within the body 22 and a spring 26b (e.g. a helical spring or any other type of spring) that is held in a compressed state by the dissolvable structure 26a. The stimulus 28 according to the example embodiment of Fig. 2 comprises a chemical (or agent) 28 that dissolves the structure 26a. The dissolution of the structure 26a causes the spring 26b to decompress, such that the pointed tip 24 is released from the end of the body 22.

In more detail, Fig. 2(a) illustrates the chemical 28 being administered, Fig. 2(b) illustrates the structure 26a being dissolved by the chemical 28, and Fig. 2(c) illustrates the pointed tip 24 being released from the end of the body 22 following the dissolution of the structure 26a by the administered chemical. Thus, according to this illustrated example embodiment, the pointed tip 24 is released from the end of the body 22 upon administration of the chemical 28 to the structure 26a. As mentioned earlier, the dissolution of the structure 26a causes the spring 26b to decompress, such that the pointed tip 24 is released from the end of the body 22. Thus, the compressed spring 26b stores energy while the structure 26a holds the pointed tip 24 within the body 22. The energy stored in the compressed spring 26b is then released when the chemical 28 dissolves the structure 26a. This release of energy is then be translated into the pointed tip 24 to release the pointed tip 24 from the end of the body 22.

In some embodiments, the dissolvable structure 26a may dissolve to a certain level of weakening, at which point the structure 26a may break due to the force of the decompression of the spring 26b to release the pointed tip from the end of the body. In some embodiments, the dissolvable structure 26a may comprise a wire, such as a central pull wire, a rod, or any other structure suitable for holding the pointed tip 24 within the body 22. The structure 26a can be made of any material suitable of being dissolved by a chemical (or agent), such as any of those materials described earlier in relation to the dissolvable cap 16 of the embodiment illustrated in Fig. 1. Similarly, the chemical (or agent) 28 can, for example, comprise any of those described earlier in relation to the chemical 18 of the embodiment illustrated in Fig. 1. The spring 26b can be made of a material that is not dissolved by the chemical 28, such as a material that is resistant to the chemical 28.

The chemical 28 may be administered in the embodiment illustrated in Fig. 2 in the same manner as described earlier with reference to the administration of the chemical 18 in the embodiment illustrated in Fig. 1. In some embodiments, the chemical 28 may itself directly dissolve the structure 26a. In other embodiments, the chemical 28 may indirectly dissolve the structure 26a. For example, in some embodiments, the body 22 may comprise an enclosure housing the structure 26a and the enclosure may comprise a fluid that can be activated by the chemical 28 to dissolve the structure 26a. The enclosure can, for example, be penetrated to allow the chemical 28 to be administered to activate the fluid housed in the enclosure. Although not illustrated, in some embodiments, the chemical 28 may comprise body fluids. In these embodiments, the body 22 may comprise an enclosure housing the structure 26a and the administration of the chemical 28 can comprise breaking or puncturing the enclosure (or a seal on the enclosure) to allow body fluids to enter the enclosure to dissolve the structure 26a.

Although not illustrated in the figures, in another embodiment, the part operable to expose the pointed tip may comprise a meltable cap (or retainer) covering the pointed tip. In this embodiment, the stimulus can induce heat in the cap that melts the cap to expose the pointed tip at the end of the body when the stimulus is applied. For example, the temperature of the cap can be increased by the heat induced by the stimulus until melting of the cap occurs. In some embodiments, the stimulus can be a heat source applied to the skin to induce heat in the cap through the skin, which melts the cap to expose the pointed tip at the end of the body. In other embodiments, the stimulus can be another energy source applied to the skin and energy from that energy source may be converted into heat in the cap, which melts the cap to expose the pointed tip at the end of the body. Examples of another energy source that may be used include, but are not limited to, a light source (such as a laser, flashlamp, or any other light source), ultrasound (such as high-intensity focused ultrasound (HIFU)), an induction coil, or any other energy source, or any combination of energy sources, suitable for inducing heat in the cap.

For example, in an embodiment where the energy source applied to the skin comprises ultrasound, the ultrasound applied to the skin can produce acoustic vibrations. These acoustic vibrations may be converted into heat in the cap, which melts the cap to expose the pointed tip at the end of the body. In an embodiment where the energy source applied to the skin comprises a light source, the light source can be configured to emit photons into the skin that can be absorbed by the cap and the absorbed photons may be converted into heat in the cap, which melts the cap to expose the pointed tip at the end of the body. In some embodiments, the cap may be formed of a material and/or have a size such that only a specific light source of a certain energy, wavelength and/or pulse duration can melt the cap (e.g. by matching absorption coefficient and thermal relaxation time of the cap). In some embodiments, the cap may be colored with a pigment that selectively absorbs light from the light source (e.g. a pigment that is not available in the skin, such as a green pigment, which absorbs red light) and subsequently converts that absorbed light into heat in the cap, which melts the cap to expose the pointed tip at the end of the body. In an embodiment where the energy source applied to the skin comprises an induction coil, the implantable device may be ferromagnetic and may comprise the induction coil. The induction coil may induce heat in the cap by electromagnetic induction, which melts the cap to expose the pointed tip at the end of the body.

Although also not illustrated in the figures, in another embodiment, the part operable to expose the pointed tip may comprise a structure holding the pointed tip within the body. For example, the structure may comprise a spring (e.g. a helical spring or any other type of spring) held in a compressed state by a bed of meltable material (e.g. fat or wax). The meltable material may, for example, be any material that is configured to melt at a temperature above body temperature. In some embodiments, the meltable material may be configured to melt at a temperature that is not too high to cause pain or discomfort to the individual. For example, in some embodiments, the meltable material may be configured to melt at a temperature in a range from 40 to 55 °C. In this embodiment, the stimulus can induce heat in the meltable material that melts the material to cause decompression of the spring to release the pointed tip from the end of the body. Thus, in this embodiment, the stimulus results in the release of stored energy in the spring and the force of the spring decompressing can be translated into the pointed tip to release the pointed tip from the end of the body. The stimulus used to induce heat in the meltable material can comprise any of those mentioned earlier. In some embodiments, the material may comprise a mixture of ferrite nanoparticles. In some embodiments, the stimulus used to induce heat in the meltable material may comprise a radiofrequency pulse. For example, the radiofrequency pulse may have a frequency of a couple of hundred kHz and may heat the material up to more than 50°C in a couple of seconds.

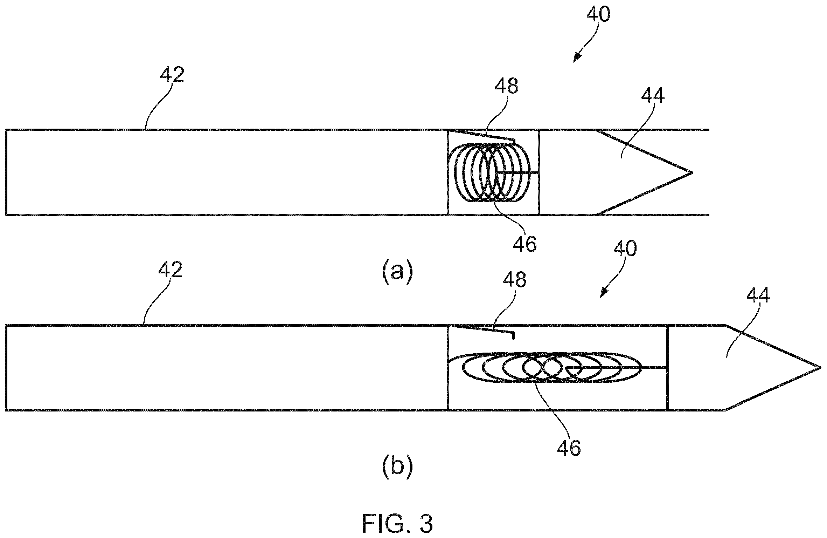

Fig. 3 illustrates an example implantable device 40 according to another embodiment. As illustrated in Fig. 3, the implantable device 40 comprises a body 42. As described earlier, the body 42 comprises a part 46 operable to expose a pointed tip 44 at an end of the body 42 upon application of a stimulus. In the illustrated example embodiment of Fig. 3, the pointed tip 44 can be stored inside the implantable device 40 (e.g. in a retracted state) prior to application of the stimulus. As also described earlier, when exposed, the pointed tip 44 is configured to enable the implantable device 40 to be moved through tissue upon application of an external force (not illustrated) to the implantable device 40.

In the illustrated example embodiment of Fig. 3, the part 46 operable to expose the pointed tip 44 comprises a spring 46 (e.g. a helical spring or any other type of spring) held in a compressed state by a mechanism 48. The mechanism 48 can take various forms. For example, in some embodiments, the mechanism 48 can comprise a lock (e.g. a form-based lock such as a pawl, a friction-based lock such as a brake, or any other type of lock), or any other type of mechanism that can hold the spring 46 in a compressed state. In the illustrated example embodiment of Fig. 3, the stimulus comprises a disengagement (or disablement) of the mechanism 48 to decompress the spring 46 to release the pointed tip 44 from the end of the body 42. In more detail, Fig. 3(a) illustrates the spring 46 held in the compressed state by the mechanism 48 and Fig. 3(b) illustrates the release of the pointed tip 44 from the end of the body 42 by decompression of the spring 46 through disengagement of the mechanism 48. Thus, energy stored in the compressed spring can be released and the force of the spring decompressing can be translated into the pointed tip 44 to release the pointed tip 44 from the end of the body 42.

In the illustrated example embodiment of Fig. 3, the mechanism 48 may be disengaged in any suitable way. For example, in some embodiments, the mechanism 48 may be mechanically disengaged (e.g. by mechanically moving the mechanism 48 away from the spring 46), chemically disengaged (e.g. by dissolving the mechanism 48), using a magnetic or electromagnetic field (e.g. that displaces the mechanism 48 away from the spring 48), or in any other suitable way. In some embodiments, the implantable device 40 may be orientated in the tissue with the mechanism 48 at the side of the external surface of the skin.

Although not illustrated in Fig. 3, in other embodiments, the part 46 operable to expose a pointed tip 44 at an end of the body 42 may comprise a swelling body (e.g. a hydrogel) as an alternative to the spring 46 and the mechanism 48. For example, the swelling body may be configured to swell upon application of a stimulus to release the pointed tip 44 from the end of the body 42.

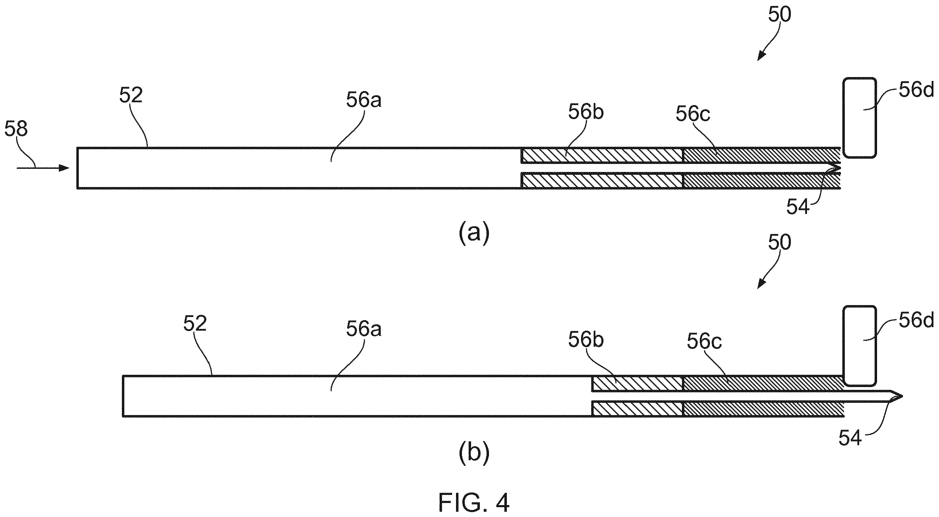

Fig. 4 illustrates an example implantable device 50 according to another embodiment. As illustrated in Fig. 4, the implantable device 50 comprises a body 52. The body 52 comprises a pointed tip carrier 56a. The pointed tip carrier 56a comprises the pointed tip 54. More specifically, the pointed tip 54 is located at an end of the pointed tip carrier 56a. Effectively, the pointed tip 54 is part of the pointed tip carrier 56a in this illustrated example embodiment. As described earlier, the body 52 comprises a part 56b, 56c operable to expose the pointed tip 54 at an end of the body 52 upon application of a stimulus 58. In the illustrated example embodiment of Fig. 4, the pointed tip 54 is stored inside the implantable device 50 (or, more specifically, covered by a part 56c of the elongate body 52) prior to application of the stimulus 58. In this way, the pointed tip 54 is protected prior to application of the stimulus 58. As also described earlier, when exposed, the pointed tip 54 is configured to enable the implantable device 50 to be moved through tissue upon application of an external force 58 to the implantable device 50.

In more detail, in the illustrated example embodiment of Fig. 4, the part 56b, 56c operable to expose the pointed tip 54 comprises a compressible portion 56b and a rigid portion 56c. The stimulus comprises a force 58 that pushes the pointed tip carrier 56a to release the pointed tip 54 from the end of the body 52. More specifically, the stimulus comprises a force 58 that pushes the pointed tip carrier 56a at an end that is opposite to the end at which the pointed tip 54 is located. In the illustrated example embodiment of Fig. 4, the stimulus for releasing the pointed tip 54 comprises the same force as the external force for moving the implantable device 50. In some embodiments, the force 58 can be a manual force. The manual force can, for example, drive out the pointed tip 54 from the end of the body 52 via movement of the pointed tip component 56a.

Fig. 4(a) and Fig. 4(b) illustrate the implantable device 50 in two different states. In more detail, Fig. 4(a) illustrates the implantable device 50 in a state at the start of application of the force 58 to push the pointed tip carrier 56a. Fig. 4(b) illustrates the implantable device 50 in a state after application of the force 58 to push the pointed tip carrier 56a, where the pointed tip 54 is released from the end of the body 52. The force 58 may be applied axially to the pointed tip carrier 56a of the body 52 of the implantable device 50 according to some embodiments.

The force 58 is applied to the pointed tip carrier 56a whilst at least a portion (e.g. one corner) of the end of the body 52 from which the pointed tip 54 is released is blocked by a blocking member 56d. The blocking member can, for example, be tissue or any other form of member that can block at least a portion of the end of the body 52. Thus, the pointed tip 54 is released from the end of the body 52 when the pointed tip carrier 56a is pushed by the force 58 simultaneously with the at least a portion of the end of the body 52 (from which the pointed tip 54 is released) being blocked by the blocking member 56d. As mentioned earlier, according to the example embodiment illustrated in Fig. 4, the body 52 of the implantable device 50 comprises a compressible portion 56b and a rigid portion 56c. Thus, when the force 58 is applied in this example embodiment, the force 58 compresses the compressible portion 56b against the rigid portion 56c to release the pointed tip 54 from the end of the body 52. In more detail, the force 58 applied to the pointed tip carrier 56a moves the pointed tip carrier 56a closer to the rigid portion 56c. The pointed tip carrier 56a thus compresses the compressible portion 56b against the rigid portion 56c.The pointed tip 54 can thus move with the pointed tip carrier 56a such that the rigid portion 56c of the body 52 of the implantable device 50 is pushed back over the pointed tip 54 to release the pointed tip 54 from the end of the body 52. In some embodiments, the compression of the compressible portion 56b may be elastic such that on release of the force 58 from the pointed tip carrier 56a, the compressible portion 56b decompresses such that the rigid portion 56c moves back over the pointed tip 54 to re-cover the pointed tip 54.

Although not illustrated in the figures, in another embodiment, the part operable to expose the pointed tip may comprise a cap (or retainer) covering the pointed tip and the stimulus may comprise a force that breaks the cap to expose the pointed tip at the end of the body when the force is applied to the cap. Thus, the stimulus may be a mechanical stimulus (e.g. a purely mechanical stimulus). For example, in some embodiments, the stimulus may comprise a force applied externally to the skin. The force can be applied directly to the skin by a person or indirectly using a tool, such as tweezers. In some embodiments, the force can break the cap by destruction of the cap.

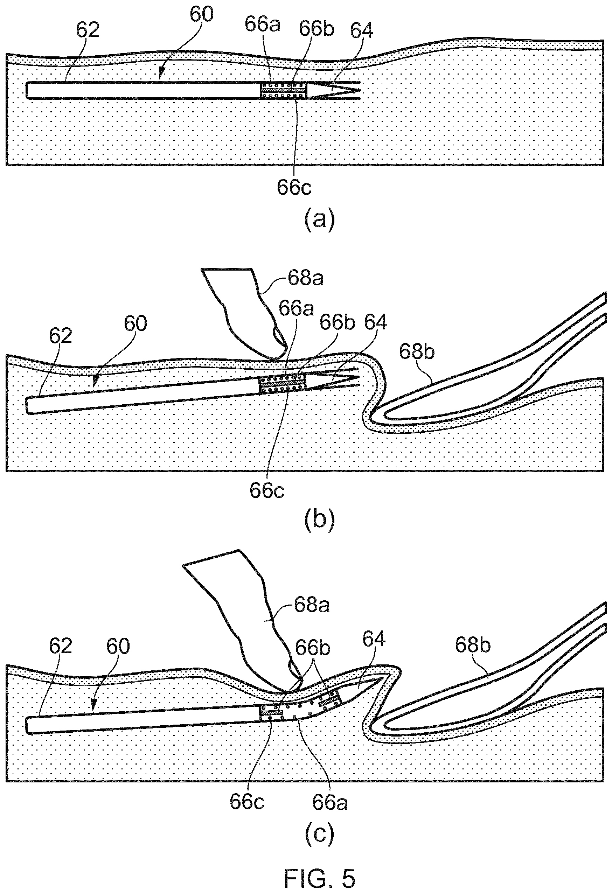

Fig. 5 illustrates an example implantable device 60 according to another embodiment. As illustrated in Fig. 5, the implantable device 60 comprises a body 62. As described earlier, the body 62 comprises a part 66a, 66b, 66c operable to expose a pointed tip 64 at an end of the body 62 upon application of a stimulus 68a. In the illustrated example embodiment of Fig. 5, the pointed tip 64 can be stored inside the implantable device 60 (e.g. in a retracted state) prior to application of the stimulus 68a. As also described earlier, when exposed, the pointed tip 64 is configured to enable the implantable device 60 to be moved through tissue upon application of an external force (not illustrated) to the implantable device 60.

In the illustrated example embodiment of Fig. 5, the part 66a, 66b, 66c operable to expose the pointed tip 64 comprises a flexible (e.g. elastic) or deformable portion 66a and the stimulus 68a comprises a force 68a that bends the flexible or deformable portion 66a to release the pointed tip 64 from the end of the body 62 when the force 68a is applied to the flexible or deformable portion 66a. For example, the force 68a can be applied using a finger or tool. Aside from the flexible or deformable portion 66a of the body 62, the rest of the body 62 (and optionally also the pointed tip 64) may be rigid. The flexible or deformable portion 66a of the body 62 can be positioned between a rigid portion of the body 62 and the pointed tip 64.

In more detail, Fig. 5(a) illustrates the implantable device 60 in the tissue prior to application of the force 68a to the flexible or deformable portion 66a, Fig. 5(b) illustrates the force 68a about to be applied to the flexible or deformable portion 66a, and Fig. 2(c) illustrates the pointed tip 64 being released from the end of the body 62 upon application of the force 68a to create a bending moment on the flexible or deformable portion 66a. In some embodiments where the implantable device 60 is to be removed from the skin, as illustrated in Fig. 5(c), an external removal device 68b may be used to aid in creating the bending moment on the flexible or deformable portion 66a and thus in removing the implantable device 60 from the skin.

In some embodiments, as illustrated in Fig. 5, the part 66a, 66b, 66c operable to expose the pointed tip 64 may also comprise a structure (or mechanism) 66b connecting the pointed tip 64 to the rigid portion of the body 62. More specifically, for example, the flexible or deformable portion 66a can comprise the structure 66b that connects the pointed tip 64 to the rigid portion of the body 62. In some embodiments, the structure 66b may comprise a wire (e.g. a central wire, such as a central pull wire), a rod (e.g. a central rod), or any other structure suitable for connecting the pointed tip 64 to the rigid portion of the body 62. In embodiments comprising the structure 66b, the force 68a applied to the flexible or deformable portion 66a that bends the flexible or deformable portion 66a can also bend the structure 66b connecting the pointed tip 64 to the rigid portion of the body 62 to release the pointed tip 64 from the end of the body 62. In some embodiments, as illustrated in Fig. 5(c), the force 68a may break the structure 66b connecting the pointed tip 64 to the rigid portion of the body 62 to release the pointed tip 64 from the end of the body 62.

The structure 66b can thus be made of any material suitable of being broken by the force 68a applied to the flexible or deformable portion 66a according to some embodiments. In some embodiments, the structure 66b may break when bent over a threshold angle. In some embodiments, a bending stiffness of the structure 66b may be such that mere pressure from outside is insufficient to bend the implantable device 60 enough to break the structure 66b. For example, in some embodiments, the structure 66b may only break when the implantable device 60 is supported (and optionally also lifted) at the pointed tip 64 by a removal device 68b (such as a spoon-like tool) that is pressed under the implantable device 60 from outside the skin. In this way, a sufficiently large moment can be built up that can lead to the threshold angle that breaks the structure 66b.

In some embodiments, as illustrated in Fig. 5, the part 66a, 66b, 66c operable to expose the pointed tip 24 can also comprise a spring 66c (e.g. a helical spring or any other type of spring) that is held in a compressed state by the structure 66b. In these embodiments, as illustrated in Fig. 5(c), the breaking of the structure 66b upon application of the force 68a to the flexible or deformable body 66a causes the spring 66b to decompress, such that the pointed tip 64 is released from the end of the body 62. Thus, in these embodiments, the compressed spring 66c stores energy while the structure 66b holds the pointed tip 64 within the body 62. The energy stored in the compressed spring 26b is then released when the force 68a breaks the structure 66b. This release of energy is then be translated into the pointed tip 64 to release the pointed tip 64 from the end of the body 62. Thus, in some embodiments, the spring 66c can push the pointed tip 64 out of the skin.

Although not illustrated in the figures, in another embodiment, the part operable to expose the pointed tip may comprise an expandable (or swelling) body and the stimulus may comprise a catalyst that causes expansion of the expandable body to release the pointed tip from the end of the body when the expandable body is exposed to the catalyst. In some embodiments, the catalyst may comprise body fluid or any other fluid (e.g. a fluid administered by a needle). In some embodiments, a closed compartment may comprise the expandable body. In these embodiments, for example, the closed compartment may be opened by puncturing the closed compartment (e.g. with a needle). The catalyst can then enter the closed compartment through an opening formed by puncturing the closed compartment, such that the expandable body is exposed to the catalyst to cause expansion (or swelling) of the expandable body to release the pointed tip from the end of the body. In other embodiments, a seal around the catalyst may be broken (e.g. by a needle or by an applied force such as bending) to release the catalyst and thus expose the expandable body to the catalyst to cause expansion (or swelling) of the expandable body to release the pointed tip from the end of the body.

Although also not illustrated in the figures, in other embodiments, there is also provided a vibrational method of triggering the exposure of the pointed tip. For example, in some embodiments, the part operable to expose the pointed tip may comprise a cap covering the pointed tip, which is tuned to fracture at a predefined frequency, and the stimulus may comprise a vibration at the predefined frequency to fracture the cap to release the pointed tip from the end of the body when the vibration is applied. In other embodiments, the part operable to expose the pointed tip may comprise a mechanism tuned to disengage at the predefined frequency and the stimulus may comprise a vibration at the predefined frequency to disengage the mechanism to release the pointed tip from the end of the body when the vibration is applied.

In these embodiments, the mechanism may be holding the pointed tip within the body and disengagement of the mechanism may then release the pointed tip from the end of the body. In some embodiments, the mechanism that is tuned to disengage at the predefined frequency may comprise a mass-spring system with a natural frequency tuned to the predefined frequency. In these embodiments, as the vibrational energy is transmitted from a source, the internal oscillation amplitude of the mass-spring system increases (e.g. rapidly) and, at or close to the predefined frequency, the mechanism holding the pointed tip within the body can be caused to disengage to release energy stored in a spring of the mass-spring system and thus release the pointed tip from the end of the body. Alternatively, at or close to the predefined frequency, a mass of the mass-spring system can hit a lever that causes the mechanism to disengage to release energy stored in the spring of the mass-spring system and thus release the pointed tip from the end of the body. In some embodiments, the mechanism may disengage at the predefined frequency by breaking at the predefined frequency to release the pointed tip from the end of the body. For example, the vibrational energy may directly or indirectly induce heat in the mechanism at the predefined frequency, which may break the mechanism.

In some embodiments, the vibration at the predefined frequency may comprise vibrational energy at the predefined frequency. The vibration may comprise one or more modes of vibration, such as any one or more of longitudinal vibrations, torsional vibrations, and bending vibrations. A source of the vibration may be placed on, applied to or coupled to an external surface of the skin above the implantable device. The position of the source of vibration relative to the implantable device may be approximate, rather than exact. In some embodiments, the source of vibration may comprise a source of ultrasound, which can be configured to produce acoustic vibrations at the predefined frequency.

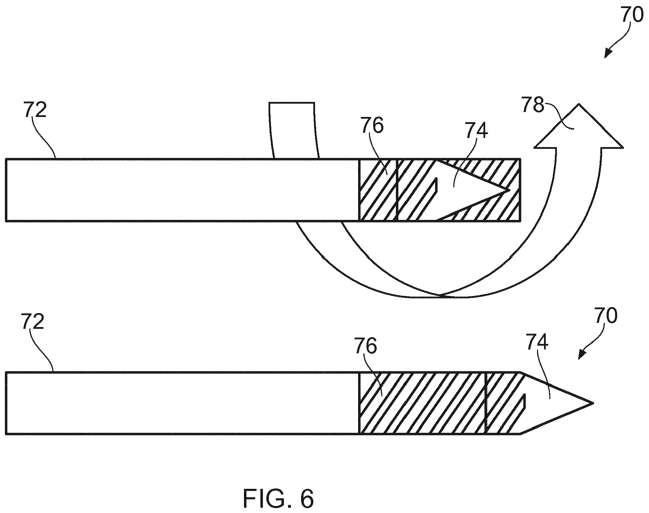

Fig. 6 illustrates an example implantable device 70 according to another embodiment. As illustrated in Fig. 6, the implantable device 70 comprises a body 72. As described earlier, the body 72 comprises a part 76 operable to expose a pointed tip 74 at an end of the body 72 upon application of a stimulus 78. In the illustrated example embodiment of Fig. 6, the pointed tip 74 can be stored inside the implantable device 70 (e.g. in a retracted state) prior to application of the stimulus 78. As also described earlier, when exposed, the pointed tip 74 is configured to enable the implantable device 70 to be moved through tissue upon application of an external force (not illustrated) to the implantable device 70. In the illustrated example embodiment of Fig. 6, the part 76 operable to expose the pointed tip 74 and the pointed tip 74 are threadably engaged and the stimulus 78 comprises a rotating magnetic field 78 that rotates the pointed tip 74 along the part 76 to release the pointed tip 74 from the end of the body 72 when the rotating magnetic field is applied. In effect, the rotating magnetic field can be used to screw out the pointed tip 74 from the end of the body 72.

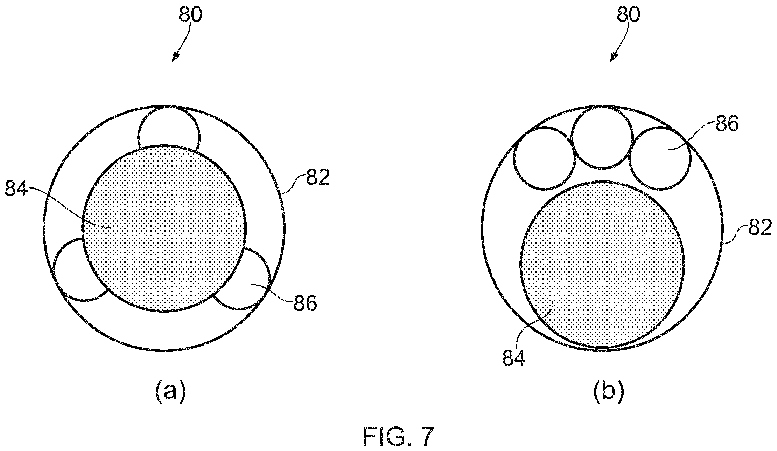

Fig. 7 illustrates an example implantable device 80 according to another embodiment. As illustrated in Fig. 7, the implantable device 80 comprises a body 82. As described earlier, the body 82 comprises a part 86 operable to expose a pointed tip 84 at an end of the body 82 upon application of a stimulus. In the illustrated example embodiment of Fig. 7, the pointed tip 84 can be stored inside the implantable device 80 (e.g. in a retracted state) prior to application of the stimulus. As also described earlier, when exposed, the pointed tip 84 is configured to enable the implantable device 80 to be moved through tissue upon application of an external force (not illustrated) to the implantable device 80.

In the illustrated example embodiment of Fig. 7, the part 86 operable to expose the pointed tip 84 comprises a plurality of magnetic structures 86 arranged around the pointed tip 84 and configured to hold the pointed tip 84 inside the implantable device 80 prior to application of the stimulus. The plurality of magnetic structures can, for example, comprise a plurality of metal balls. In these embodiments, the stimulus comprises a magnetic field that attracts the plurality of magnetic structures to the same location around (e.g. the same side of) the pointed tip 84 to release the pointed tip 84 from the end of the body 82 when the magnetic field is applied. Fig. 7(a) illustrates the arrangement of the plurality of magnetic structures 86 around the pointed tip 84 when there is no magnetic field applied and Fig. 7(b) illustrates the arrangement of the plurality of magnetic structures 86 around the pointed tip 84 upon application of the magnetic field. The movement of the plurality of magnetic structures 86 according to the illustrated example embodiment of Fig. 7 is independent of the orientation of the implantable device 80.

As illustrated in Fig. 7(a), in some embodiments, the plurality of magnetic structures 86 may be arranged around the pointed tip 84 at a constant distance from each other when there is no magnetic field applied. For example, the plurality of magnetic structures 86 may be arranged symmetrically around the pointed tip 84 when there is no magnetic field applied. As illustrated in Fig. 7(b), upon application of a magnetic field, the movement of the plurality of magnetic structures 86 toward the applied magnetic field reduces the distance between each of the plurality of magnetic structures 86. For example, the plurality of magnetic structures 86 may be arranged asymmetrically around the pointed tip 84 upon application of the magnetic field. In effect, the plurality of magnetic structures 86 collect at the side of the body 82 at a location that is closer to the stratum corneum, where the magnetic field is being applied to an external surface of the skin. This enlarges the area in which the pointed tip 84 can move to allow passage of the pointed tip 84 through the body 82 such that the pointed tip 84 can be released from the end of the body 82 when the magnetic field is applied.

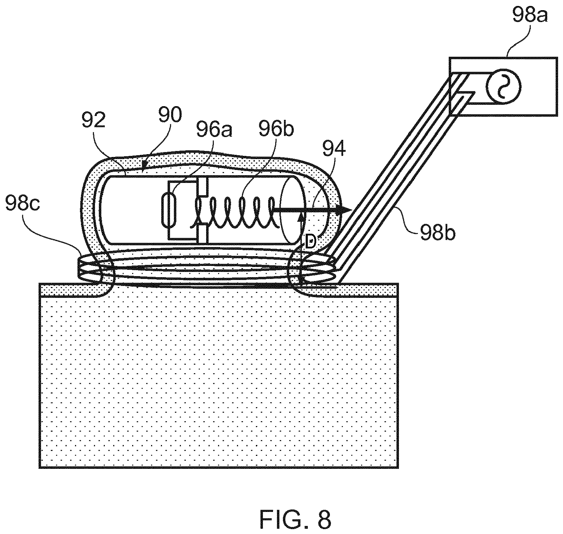

In another embodiment, the structures 86 of Fig. 7 may instead be moveable in the earlier described manner upon application of an electric field (rather than a magnetic field). For example, the structures 86 may all have a positive charge or may all have a negative charge prior to application of the electric field to be in the arrangement illustrated in and described with reference Fig. 7(a). Upon application of the electric field, the charge may be removed from the structures to cause the structures to move into the arrangement illustrated in and described with reference to Fig. 7(b). Thus, the pointed tip can be released from the end of the body 82 when the electric field is applied. In other embodiments, the stimulus that moves the structures 86 of Fig. 7 may be a mechanical force. In the embodiments where the structures 86 are moved upon application of an electric field or mechanical force, the structures 86 may or may not be magnetic. In other embodiments, the part operable to expose the pointed tip may comprise a component configured for activation by magnetic field, electromagnetic field or radiofrequency (RF) signal and the stimulus may comprise the magnetic field, electromagnetic field or radiofrequency (RF) signal that activates the component to release the pointed tip from the end of the body when the magnetic field, electromagnetic field or radiofrequency (RF) signal is applied. Figs. 8, 9 and 10 illustrate examples of these embodiments.

Fig. 8 illustrates an example implantable device 90 according to an embodiment. As illustrated in Fig. 8, the implantable device 90 comprises a body 92. As described earlier, the body 92 comprises a part 96a, 96b operable to expose a pointed tip 94 at an end of the body 92 upon application of a stimulus. In the illustrated example embodiment of Fig. 8, the pointed tip 94 can be stored inside the implantable device 90 (e.g. in a retracted state) prior to application of the stimulus. As also described earlier, when exposed, the pointed tip 94 is configured to enable the implantable device 90 to be moved through tissue upon application of an external force (not illustrated) to the implantable device 90.

In the illustrated example embodiment of Fig. 8, the part 96a, 96b operable to expose the pointed tip 94 comprises a component configured for activation by an electromagnetic field. The part 96a, 96b operable to expose the pointed tip 94 can, for example, comprise an electro-magnetic trigger mechanism that is activated by an electromagnetic field. More specifically, the part 96a, 96b operable to expose the pointed tip 94 in the illustrated example embodiment of Fig. 8 comprises a switch 96a (e.g. a reed switch or any other type of switch) and a spring 96b (e.g. a helical spring or any other type of spring) configured for activation by an electromagnetic field. In some embodiments, the switch 96a can respond reversibly to the electromagnetic field. The stimulus comprises an electromagnetic field that activates the switch 96a and spring 96b to release the pointed tip 94 from the end of the body 92 when the electromagnetic field is applied.

In more detail, in the illustrated example embodiment of Fig. 8, the switch 96a is electrically connected to the spring 96b. The spring 96b is operably engaged with the pointed tip 94. The spring 96b may, for example, be coupled to the pointed tip 94, connected to the pointed tip 94, attached to the pointed tip 94, or engaged with the pointed tip 94 in any other way that enables the spring 96b to operate the pointed tip 94 in the manner described. The spring 96b is under preload in the absence of the electromagnetic field. Upon application of the electromagnetic field (e.g. when the electromagnetic field is induced in the implantable device 90 through the skin), the switch 96a is caused to close by the electromagnetic field and this in turn causes the spring 96b to extend (or stretch) to release the pointed tip 94 from the end of the body 92.

In the illustrated example embodiment of Fig. 8, the electromagnetic field may be induced by way of any device (or component) that is configured to generate an electromagnetic field. In some embodiments, such as that illustrated in Fig. 8, the device configured to generate the electromagnetic field may comprise an integrated alternating current (AC) source or a radiofrequency (RF) oscillator 98a and a metal lasso (or metal sling) 98b, 98c. A metal lasso 98b, 98c is an element that comprises an elongate portion 98b with a metal loop 98c at an end of the elongate portion 98b. The metal loop 98c may also be referred to as a field guidance element. The metal loop 98c may, for example, be placed around (or under) the implantable device 90 through the skin. In this way, the metal loop 98c may be used to grasp and lift the implantable device 90 from outside the skin. In effect, the metal loop 98c forms an inductive loop around the implantable device 90. Thus, in the illustrated example embodiment of Fig. 8, an electromagnetic field is induced in the implantable device 90 through the use of the metal loop 98c of the metal lasso. The elongate portion 98b and/or the metal loop 98c of the metal lasso can be insulated (e.g. plastic covered).

In some embodiments, as illustrated in Fig. 8, the metal loop 98c may comprise one or more inductive loops (or windings). The one or more inductive loops may, for example, be vertically arranged. Although not illustrated in Fig. 8, in some embodiments, the one or more inductive loops 98c may be wrapped around a (e.g. flexible) hollow core. The hollow core may, for example, be made of a ferroelectric material (e.g. iron). In this way, the electromagnetic field induced in the implantable device 92 can be increased. Moreover, according to this embodiment, it is still possible to pull the metal loop 98c comprising the one or more inductive loops tightly around (or under) the implantable device 90 from outside the skin to grasp and lift the implantable device 90. Although an embodiment is described with a hollow core, it will be understood that there may be no hollow core in other embodiments. In some embodiments, as illustrated in Fig. 8, a distance D between the metal loop 98c and a center of the implantable device 90 may be small (e.g. less than 5 millimeters).

According to some embodiments involving the application of an electromagnetic field, the device configured to generate the electromagnetic field may be manually switched on (e.g. on-demand). In some embodiments, for example, the electromagnetic field may be controllable via a switch (or button), which can be activated to turn on and/or turn off the electromagnetic field. The switch (or button) may be provided on the device that is configured to generate an electromagnetic field. In the illustrated embodiment of Fig. 8, for example, the switch (or button) may be provided on the metal lasso 98b, 98c or the alternating current (AC) source or a radiofrequency (RF) oscillator 98a. In this way, the unintentional activation of the pointed tip 94 before the metal loop 98c is brought into the correct position around (or under) the implantable device 90 can be avoided. Although the illustrated example embodiment of Fig. 8 has been described with reference to an induced electromagnetic field, it will be understood that the same embodiment can be achieved through use of a (e.g. permanent) magnetic field instead of the induced electromagnetic field.

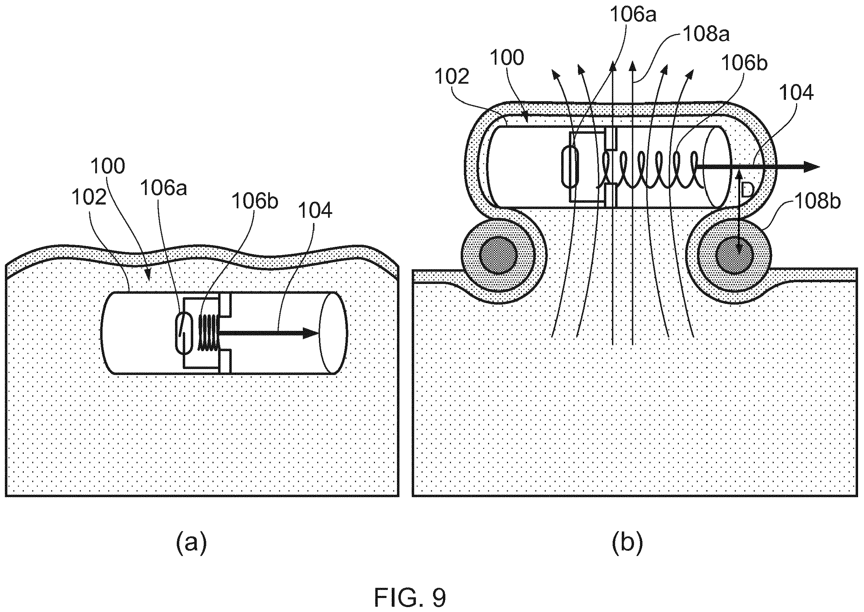

Fig. 9 illustrates an example implantable device 100 according to another embodiment. As illustrated in Fig. 9, the implantable device 100 comprises a body 102. As described earlier, the body 102 comprises a part 106a, 106b operable to expose a pointed tip 104 at an end of the body 102 upon application of a stimulus 108a. In the illustrated example embodiment of Fig. 9, the pointed tip 104 can be stored inside the implantable device 100 (e.g. in a retracted state) prior to application of the stimulus 108a. As also described earlier, when exposed, the pointed tip 104 is configured to enable the implantable device 100 to be moved through tissue upon application of an external force (not illustrated) to the implantable device 100.

In the illustrated example embodiment of Fig. 9, the part 106a, 106b operable to expose the pointed tip 104 comprises a component configured for activation by an (electro-) magnetic field. More specifically, the part 106a, 106b operable to expose the pointed tip 104 comprises a switch 106a (e.g. a reed switch or any other type of switch) and a spring 106b (e.g. a helical spring or any other type of spring) configured for activation by a (electro-) magnetic field. In some embodiments, the switch 106a can respond reversibly to the (electro-) magnetic field. The stimulus 108a comprises the (electro-) magnetic field 108a that activates the switch 106a and spring 106b to release the pointed tip 104 from the end of the body 102 when the (electro-) magnetic field 108a is applied.

In more detail, in the illustrated example embodiment of Fig. 9, the switch 106a is electrically connected to the spring 106b. The spring 106b is operably engaged with the pointed tip 104. The spring 106b may, for example, be coupled to the pointed tip 104, connected to the pointed tip 104, attached to the pointed tip 104, or engaged with the pointed tip 104 in any other way that enables the spring 106b to operate the pointed tip 104 in the manner described. The spring 106b is under pre-tension in the absence of the (electro-) magnetic field 108a. Upon application of the (electro-) magnetic field 108a (e.g. when the (electro-) magnetic field 108a is induced in the implantable device 100 through the skin), the switch 106a is caused to close by the (electro-) magnetic field 108a and this in turn causes the spring 106b to extend (or stretch) to release the pointed tip 104 from the end of the body 102.

In the illustrated example embodiment of Fig. 9, the (electro-) magnetic field 108a can be induced in the implantable device 100 through the use of a metal loop (e.g. a metal sling or a metal lasso) 108b. The metal loop 108b may, for example, be placed around (or under) the implantable device 100 through the skin. In this way, the metal loop 108b may be used to grasp and lift the implantable device 100 through the skin. The metal loop 108b can be insulated (e.g. plastic covered). In effect, the metal loop 108b forms a magnetic loop around the implantable device 100. In some embodiments, the metal loop 108b may comprise one or more permanent magnets (not illustrated). In some embodiments, the one or more permanent magnets may be flexible. In some embodiments, the one or more permanent magnets may be arranged in a predefined pattern. The one or more permanent magnets can enable a passive magnetic activation of the switch 106a, which in turn activates the spring 106b, without the use of an external electrical power source, such as an alternating current (AC) source or radiofrequency (RF) source. In some embodiments, as illustrated in Fig. 9, a distance D between the metal loop 108b and a center of the implantable device 100 may be small (e.g. less than 5 millimeters).

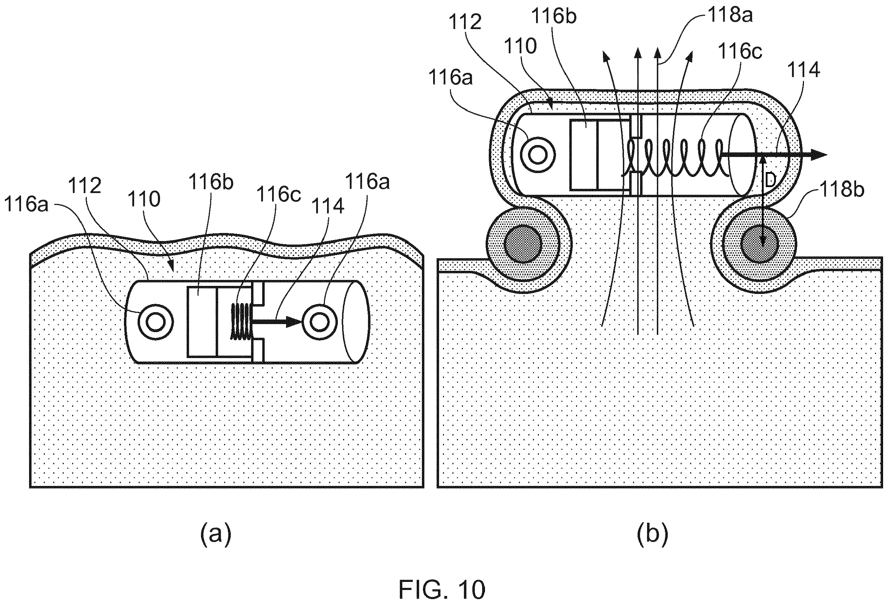

Fig. 10 illustrates an example implantable device 110 according to another embodiment. As illustrated in Fig. 10, the implantable device 110 comprises a body 112. As described earlier, the body 112 comprises a part 116a, 116b, 116c operable to expose a pointed tip 114 at an end of the body 112 upon application of a stimulus 118a. In the illustrated example embodiment of Fig. 10, the pointed tip 114 can be stored inside the implantable device 110 (e.g. in a retracted state) prior to application of the stimulus 118a. As also described earlier, when exposed, the pointed tip 114 is configured to enable the implantable device 110 to be moved through tissue upon application of an external force (not illustrated) to the implantable device 110.

In the illustrated example embodiment of Fig. 10, the part 116a, 116b, 116c operable to expose the pointed tip 114 comprises a component configured for activation by a radiofrequency (RF) signal, such as a radiofrequency (RF) wake-up signal. More specifically, the part 116a, 116b, 116c operable to expose the pointed tip 114 comprises a secondary coil 116a, a controller (e.g. a microcontroller (MCU)) 116b and a spring 116c (e.g. a helical spring or any other type of spring) configured for activation by a radiofrequency signal (e.g. a radiofrequency wake-up signal). The stimulus 118a comprises the radiofrequency signal 118a that activates the secondary coil 116a, controller 116b and spring 116c to release the pointed tip 114 from the end of the body 112 when the radiofrequency signal 118a is applied.

In more detail, in the illustrated example embodiment of Fig. 10, the controller 116b is electrically connected to the spring 116c. The spring 116c is operably engaged with the pointed tip 114. The spring 116c may, for example, be coupled to the pointed tip 114, connected to the pointed tip 114, attached to the pointed tip 114, or engaged with the pointed tip 114 in any other way that enables the spring 116c to operate the pointed tip 114 in the manner described. The spring 116c is under pre-tension in the absence of the radiofrequency signal 118a. Upon application of the radiofrequency signal 118a (e.g. when the (electro-) magnetic field 118a is transmitted to the implantable device 110 through the skin), the secondary coil 116a picks up the radiofrequency signal 118a and activates the spring 116c via the controller 116b to extend (or stretch) to release the pointed tip 114 from the end of the body 112.

In the illustrated example embodiment of Fig. 10, the radiofrequency signal 118a can be transmitted to the implantable device 110 through the use of a primary coil 118b. The primary coil 118b can thus act as a radiofrequency transmitter. The primary coil 118b may, for example, be placed around (or under) the implantable device 110 through the skin. In this way, the primary coil 118b may be used to grasp and lift the implantable device 110 from outside the skin. In this way, the implantable device 110 can be brought toward the surface of the skin. In effect, the primary coil 118b forms an inductive loop around the implantable device 110 that acts as a radiofrequency transmitter. The secondary coil 116a of the implantable device 110 picks up the radiofrequency signal 118a transmitted from the primary coil 118b and activates the spring 116c via the controller 116b to extend (or stretch) to release the pointed tip 114 from the end of the body 112, as described earlier. In some embodiments, the primary coil 118b can be insulated (e.g. plastic covered).

In some embodiments, the secondary coil 116a of the body 112 of the implantable device 110 and the primary coil 118b can be arranged in parallel to each other. In this way, more efficient coupling is achieved and thus less power is required (since the higher the coupling factor, the less input power is needed). In some embodiments, as illustrated in Fig. 10, a distance D between the primary coil 118b and a center of the implantable device 110 may be small (e.g. less than 5 millimeters). In these embodiments, a large range communication frequency (e.g. of the order of MHz to GHz, such as near-field communication (NFC) at 13.56 MHz or Bluetooth at 2.4 GHz) may be used to activate the pointed tip 114. These frequencies may be less critical to coil misalignment. Although not illustrated in Fig. 10, in some embodiments, the implantable device 110 may comprise a radiofrequency identification (RFID) chip. For example, the RFID chip may be integrated in the secondary coil 116a according to some embodiments. In this way, the implantable device 110 can be identified, e.g. to ensure the correct implantable device is removed.

In some embodiments, the primary coil 118b may be controllable via a switch (or button), which can be activated to turn on and/or turn off the transmission of the radiofrequency signal 118a. The switch (or button) may, for example, be provided on the primary coil 118b. The switch (or button) may be activated (e.g. by an operator) to turn on the transmission of the radiofrequency signal 118a once the implantable device 110 is grasped correctly. In this way, the unintentional activation of the pointed tip 114 before the primary coil 118b is brought into the correct position around (or under) the implantable device 110 can be avoided.

Although not illustrated in the figures, in any of the embodiments described herein, the part of the body of the implantable device that is operable to expose the pointed tip at an end of the body may comprise a locking mechanism. The locking mechanism can be configured to lock the pointed tip in an exposed state and prevent the pointed tip from retracting back into a retracted state (e.g. inside the body) when the applied stimulus is removed. Alternatively or in addition, in some embodiments, the body of the implantable device may comprise one or more barbs (e.g. spikes) arranged to allow the implantable device to move in one direction and prevent the implantable device moving in another (e.g. the opposite) direction.

In addition to an implantable device, as provided in this disclosure, there is provided a manipulation device (or tool) for use with the implantable device and a removal device (or tool) for use with the implantable device. There is also provided a system comprising the implantable device (as described herein) and the manipulation device (as described herein) and/or the removal device (as described herein).

The manipulation device referred to herein is a device configured for use in manipulating (e.g. moving) the implantable device within the tissue. The manipulation device can be any type of device that can be used to manipulate (e.g. move) the implantable device within the tissue. For example, the manipulation device can comprise tweezers or forceps. The removal device referred to herein is a device configured for use in removing the implantable device from the skin. For example, the pointed tip of the implantable device can puncture the stratum corneum of the skin and a removal device can be used to move the implantable device (further) through and even completely out of the stratum corneum of the skin, such as by grasping, gripping or holding the implantable device and then using a pulling or pushing force on the implantable device. The removal device can be any type of device that can be used to remove the implantable device from the skin. For example, the removal device can comprise tweezers or forceps.

In some embodiments, the manipulation device and/or the removal device can facilitate in keeping the implantable device orientated in the correct direction while enabling manipulation and/or removal of the implantable device. In some embodiments, the manipulation device and/or the removal device can be configured (e.g. shaped to) grasp, grip or hold an end of the body of the implantable device through the skin or, more specifically, grasp, grip or hold the end of the body that is opposite to the end at which the pointed tip is exposed.

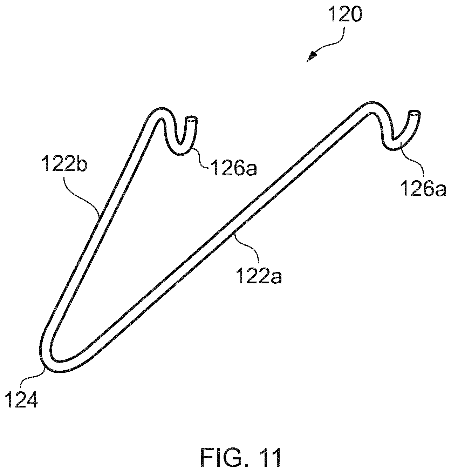

Fig. 11 illustrates an example of such a manipulation and/or the removal device 120 according to an embodiment. In the illustrated example embodiment, the manipulation and/or the removal device 120 is U-shaped or V-shaped. For example, the manipulation and/or the removal device 120 can comprise two elongate arms 122a, 122b joined by a curved or pointed portion 124. In some embodiments, the two elongate arms 122a, 122b and curved or pointed portion 124 may be flexible. In some embodiments, the ends of each of the two elongate arms 122a, 122b may be shaped in an arc 126a, 126b. In some embodiments, the ends of each of the two elongate arms 122a, 122b may be rigid (or stiff).

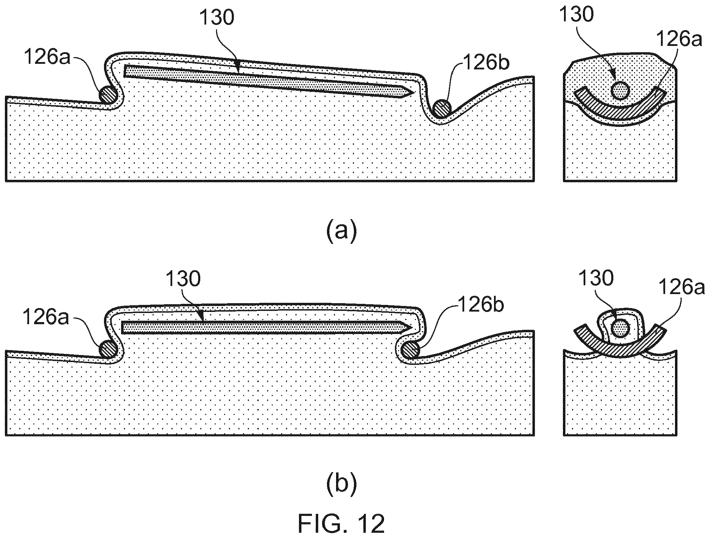

Fig. 12 illustrates an example of the manipulation and/or the removal device 120 in use according to an embodiment. In the illustrated example embodiment, the manipulation and/or the removal device 120 can be configured to stabilize and/or lift an implantable device 130. As illustrated in Fig. 12(a), the arcs 126a, 126b at the ends of each of the two elongate arms 122a, 122b of the manipulation and/or the removal device 120 are positioned close to the two ends of the implantable device 130. As also illustrated in Fig. 12(a), the arcs 126a, 126b maybe positioned at sufficient distance from the two ends of the implantable device 130 to allow two dimples to be pressed locally in the skin.

Once positioned in the manner illustrated in Fig. 12(a), the manipulation and/or the removal device 120 may then be compressed as illustrated in Fig. 12(b). More specifically, as illustrated in Fig. 12(b), the arcs 126a, 126b at the ends of each of the two elongate arms 122a, 122b may be brought closer together. In this way, the implantable device 130 can be gripped (e.g. stabilized) and lifted. In some embodiments, the pointed tip may be exposed prior to the use of the manipulation and/or the removal device 120. In other embodiments, the pointed tip may be exposed subsequent to the use of the manipulation and/or the removal device 120.

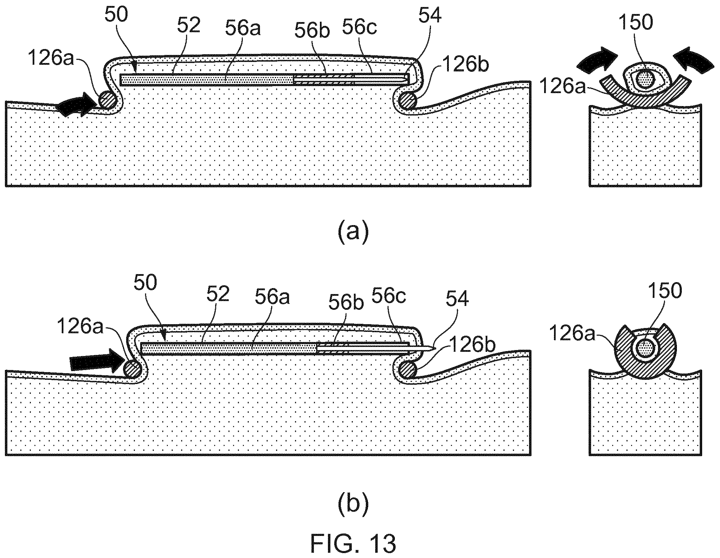

Fig. 13 illustrates an example of the manipulation and/or the removal device 120 in use according to another embodiment. In the illustrated example embodiment, the manipulation and/or the removal device 120 can be configured to stabilize and/or lift the implantable device 50 described earlier with reference to Fig. 4. The pointed tip 54 of the implantable device 50 is initially in a retracted state inside the body 52. As illustrated in Fig. 13(a), the arcs 126a, 126b at the ends of each of the two elongate arms 122a, 122b of the manipulation and/or the removal device 120 are positioned close to the two ends of the implantable device 50. As also illustrated in Fig. 13(a), the arcs 126a, 126b may be positioned at sufficient distance from the two ends of the implantable device 50 to allow two dimples to be pressed locally in the skin.

Once positioned in the manner illustrated in Fig. 13(a), the manipulation and/or the removal device 120 may then be compressed as illustrated in Fig. 13(b). More specifically, as illustrated in Fig. 13(b), the arcs 126a, 126b at the ends of each of the two elongate arms 122a, 122b may be brought closer together. In this way, the implantable device 130 can be gripped (e.g. stabilized) and lifted. In this illustrated embodiment, the pointed tip 54 of the implantable device 50 is exposed subsequent to the use of the manipulation and/or the removal device 120 in the manner described earlier with reference to Fig. 4.

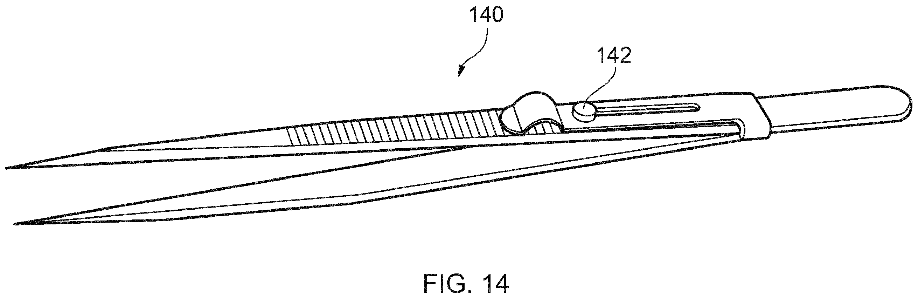

As illustrated in Fig. 13(b), according to this illustrated example embodiment, the exposed pointed tip 54 punctures the skin upon application of an external force to the implantable device 50. More specifically, the exposed pointed tip 54 punctures the skin upon continued compression of the manipulation and/or the removal device 120. Although not illustrated in Fig. 13, in other embodiments, the same mechanism can be used to move the implantable device 50 in an axial direction under the surface of the skin, e.g. in multiple steps. In some of these embodiments, the pointed tip 54 may retract back inside the body 52 in between the steps. In some embodiments, the manipulation and/or the removal device described herein may comprise a lock configured to fix the manipulation and/or the removal device in the exposed position as illustrated in Fig. 12(b) and Fig. 13(b). Fig. 14 illustrates an example of a manipulation and/or the removal device 140 comprising such a lock 142 according to an embodiment.

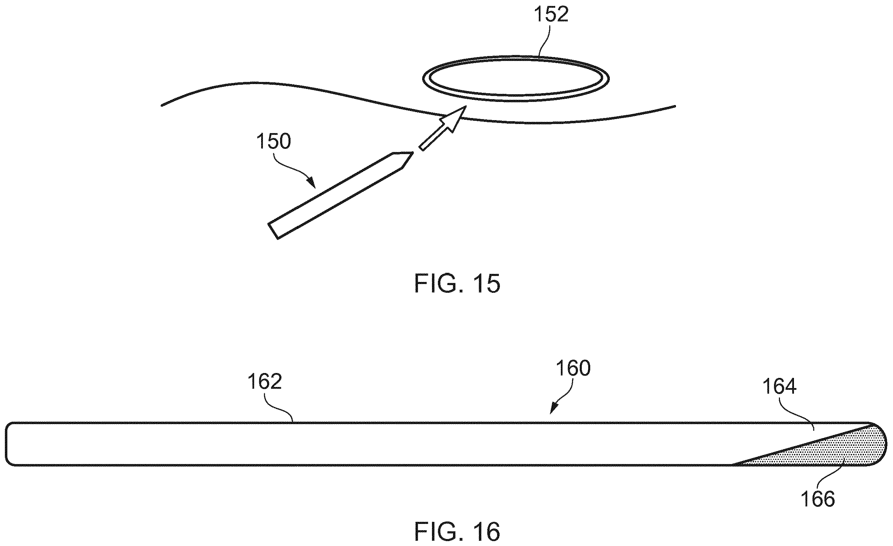

Fig. 15 illustrates an example of a manipulation and/or the removal device 152 in use according to another embodiment. In the illustrated example embodiment, the manipulation and/or the removal device 152 can be configured to provide a magnetic field above the external surface of the skin. The implantable device 150 can be attracted to the magnetic field. For example, the implantable device 150 may comprise or be made from a permanent magnet or magnetite. In some embodiments, only the pointed tip of the implantable device 150 may comprise or be made from a permanent magnet or magnetite, such that the pointed tip is attracted to the external magnetic field to allow the pointed tip to be moved to the surface of the skin for removal. In embodiments where the manipulation and/or the removal device 152 is configured to provide a magnetic field, it is possible to move the implantable device 150 through tissue without contacting the skin.

Although the pointed tip of the implantable device described herein has thus far been illustrated as having a symmetrical shape, it will be understood that the pointed tip may instead have an asymmetrical shape according to some embodiments. Fig. 16 illustrates an example of an implantable device 160 comprising a body 162, where the body 162 comprises a pointed tip 164 having an asymmetrical shape. As illustrated in Fig. 16, the part operable to expose the asymmetrical pointed tip 164 at an end of the body 162 upon application of a stimulus may comprise a cap 166, such as any of those described earlier. In other embodiments, the part may comprise any other part operable to expose the asymmetrical pointed tip 164 at the end of the body 162 upon application of a stimulus, such as any of those parts described earlier. Generally, the pointed tip described herein may have any shape that is suitable for allowing the pointed tip to move through tissue.

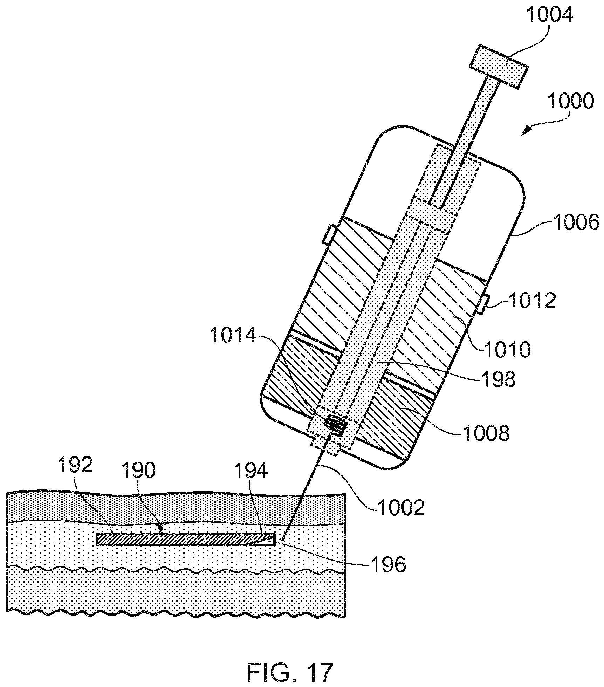

Fig. 17 illustrates an example of a manipulation and/or the removal device 1000 in use with an implantable device 190 according to another embodiment. The implantable device 190 according to this illustrated example embodiment comprises a body 192, which comprises a pointed tip 194. The pointed tip 194 is covered by a dissolvable cap 196. According to this illustrated example embodiment, the manipulation and/or the removal device 1000 is configured to apply the stimulus upon application of which the pointed tip 194 is exposed at the end of the body 192 and the external force upon application of which the implantable device 190 can be moved through tissue.

In more detail, the manipulation and/or the removal device 1000 may comprise a stimulus application system 1002, 1004, 1014 operable to apply the stimulus 198 and also an external force controller 1010 operable to apply the external force. According to this illustrated example embodiment, the stimulus 198 is the administration of a chemical (e.g. an anesthetic and/or solvent) that is suitable for dissolving the dissolvable cap 196 in the manner described earlier and the external force is a magnetic field. In some embodiments, a housing 1006 may comprise the external force controller 1010 and at least part of the stimulus application system 1002, 1004, 1014.

In some embodiments, the stimulus application system 1002, 1004, 1014 may comprise a syringe having a needle 1002 for insertion into the skin and a plunger 1004 for pushing the chemical into the skin via the needle 1002. The needle 1002 may be retractable according to some embodiments. For example, in some embodiments, the needle 1002 may be retractable via a spring-loaded mechanism 1014. In these embodiments, the needle 1002 may be extended when the stimulus application system 1002, 1004, 1014 is administering the chemical 198 to dissolve the cap 196. Similarly, the needle 1002 may be retracted when the stimulus application system 1002, 1004, 1014 is not administering the chemical 198 to dissolve the cap 196.

In some embodiments, the manipulation and/or the removal device 1000 can comprise an electromagnet 1008 and the external force controller 1010 can be configured to control the electromagnet 1008 to apply the magnetic field. The external force controller 1010 may be configured to control the electromagnet 1008 to apply a magnetic field of variable strength, e.g. from weaker to stronger. For example, the external force controller 1010 may be configured to control the electromagnet 1008 to apply a weaker magnetic field for movement of the implantable device 190 within the tissue and a stronger magnetic field for removal of the implantable device 190 from the tissue. The manipulation and/or the removal device 1000 may comprise a button 1012 that can be used to operate the external force controller 1010 in the manner described.

In some embodiments, the pointed tip 194 of the body 192 of the implantable device 190 maybe formed of a magnetic material, while the rest of the body 192 may be formed of a non-magnetic material. Thus, the pointed tip 194 will be attracted to the applied magnetic field such that the orientation of the implantable device 190, which may have changed since insertion of the implantable device 190 in the tissue, is irrelevant. As such, a user (e.g. a doctor) does not need to have knowledge of where to inject the needle 1002 of the stimulus application system 1002, 1004, 1014 prior to using the manipulation and/or the removal device 1000. Instead, this can be accomplished through the use of the manipulation and/or the removal device 1000. For example, the orientation of the implantable device 190 may be determined by controlling the electromagnet 1008 via the external force controller 1010 to apply a weak magnetic field such that the pointed tip 194 of the implantable device 190 is drawn closer to the skin surface by the electromagnet 1008. Then, the plunger 1004 of the stimulus application system 1002, 1004, 1014 can be used to administer the chemical via the needle 1002 of the stimulus application system 1002, 1004, 1014 at a location on the skin that is near the pointed tip 194 of the implantable device 190. The cap 196 is dissolved by the administered chemical to expose the pointed tip 194. The electromagnet 1008 may then be controlled via the external force controller 1010 to apply a stronger magnetic field to move the implantable device 190 through tissue, such as to pull the implantable device 190 out through the skin, using the exposed pointed tip 194.

As mentioned earlier, in some embodiments, the exposure of the pointed tip at the end of the body of the implantable device described herein can be reversible.

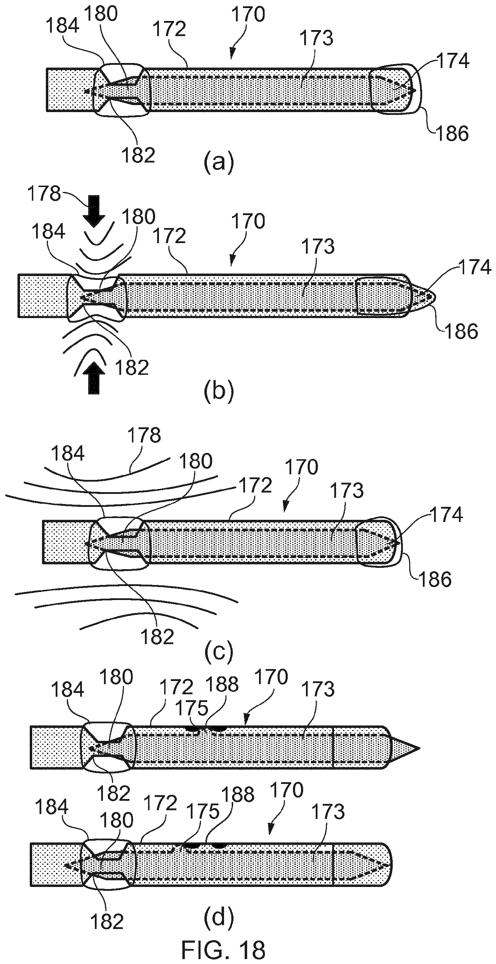

Fig. 18 illustrates an example of an implantable device 170 where the exposure of the pointed tip 174 at the end of the body 172 of the implantable device 170 is reversible. Fig. 18(a) illustrates the basic structure of the implantable device 170. As illustrated in Fig. 18(a), the body 172 of the implantable device 170 comprises a pointed tip carrier 173. The pointed tip carrier 173 comprises the pointed tip 174. More specifically, the pointed tip 174 is located at an end of the pointed tip carrier 173. Effectively, the pointed tip 174 is part of the pointed tip carrier 173 in this illustrated example embodiment. The end 180 of the pointed tip carrier 173 that is opposite to the pointed tip 174 is conical in shape in this illustrated example embodiment. The body 172 (inside which the pointed tip carrier 173 is provided) comprises a notch 182 at the conically shaped end 180 of the pointed tip carrier 173. The notch 182 is covered/filled with a flexible material (such as silicon) 184. In this illustrated example embodiment, the part of the body 172 operable to expose the pointed tip 174 at an end of the body 172 upon application of a stimulus comprises the notch 182, which is covered/filled with the flexible material 184.

According to this illustrated example embodiment of Fig. 18, the end of the body 172 from which the pointed tip 174 is released comprises a cap 186. The cap 186 is positioned to cover the pointed tip 174. The cap 186 can be flexible, e.g. comprised of a flexible material (such as silicon). In some embodiments, the cap 186 may keep interstitial fluid and encapsulation tissue from entering the body 172 of the implantable device 170. In some embodiments, the cap 186 may provide sufficient elasticity and strength when the pointed tip 174 is exposed at the end of the body 172. The body 172 according this illustrated example embodiment may be rigid, e.g. comprised of a hard material.

As illustrated in Fig. 18(b) and Fig. 18(c), the pointed tip 174 according to this illustrated example embodiment is exposed upon application of a stimulus in the form of a force 178 that squeezes the implantable device 170 at a location that causes a pressure that is sufficient to push the pointed tip carrier 173 to expose the pointed tip 174 at the end of the body 172. In the illustrated example embodiment of Fig. 18, this location is the notch 182 of the body 172, which is covered/filled with the flexible material 184. Fig. 18(b) illustrates the (targeted) force 178 applied by a tool (such as tweezers) and Fig. 18(c) illustrates the (non-targeted) force 178 applied by fingers. As illustrated, it is possible to expose the pointed tip 174 more through application of a force 178 by a tool than through application of a force 178 by fingers. In effect, the force 178 induces a (e.g. relatively local) pressure on the conically shaped end 180 of the pointed tip carrier 173, which causes a horizontal force to expose (e.g. extrude) the pointed tip 174 at the end of the body 172.

In some embodiments, although not illustrated in Fig. 18, the cap 186 may be equipped with one or more relief structures (e.g. bumps) that are pronounced when the pointed tip 174 is in a retracted state inside the body 172 to aid in preventing migration of the implantable device 170. In some embodiments, the cap 186 may then be smoothed during stretching due to the exposure of the pointed tip 174 at the end of the body 172. For example, stretching the cap 186 also stretches the one or more relief structures. The stretching of the one or more relief structures elongates them and (e.g. largely or completely) flattens them. In this way, the one or more relief structures of the cap 186 can be minimized during exposure of the pointed tip 174 to allow the pointed tip 174 to move more smoothly through tissue.

As illustrated in Fig. 18(d), in some embodiments, a body of the pointed tip carrier 173 can comprise one or more raised portions (e.g. bumps) 175. The one or more raised portions 175 may fit within the internal shape of the surrounding body 172. The one or more raised portions 175 can hold the pointed tip 174 in an exposed state. For example, as illustrated in the example embodiment of Fig. 18, the one or more raised portions 175 may fit within a recess 188 in the internal surface of the body 172 to hold the pointed tip 174 in the exposed state. In this way, the pointed tip 174 may remain in the exposed state even after removal of the stimulus 178, which is the force in this illustrated example embodiment. As illustrated in Fig. 18(d), the pointed tip 174 may still be pushed back into the retracted state. For example, the pointed tip 174 may be pushed back into the retracted state by a force (e.g. enacted by a tool, such as tweezers) applied at the pointed tip 174 and a counteracting force applied at the opposite end of the body 172 (e.g. with fingers).

There is thus provided herein an improvement that overcomes existing problems and, more specifically, an implantable (or insertable, or subcutaneous) device that overcomes existing problems. Any one or more of the embodiments described herein may be combined.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An implantable device (10, 20, 40, 50, 60, 70, 80, 90, 100, 110, 130, 150, 160, 170, 190) comprising:
a body (12, 22, 42, 52, 62, 72, 82, 92, 102, 112, 162, 172, 192) comprising a part (16, 26a, 26b, 46, 56a, 66a, 66b, 66c, 76, 86, 96a, 96b, 106a, 106b, 116a, 116b, 116c, 166, 182, 184, 196) operable to expose a pointed tip (14, 24, 44, 54, 64, 74, 84, 94, 104, 114, 164, 174, 194) at an end of the body (12, 22, 42, 52, 62, 72, 82, 92, 102, 112, 162, 172, 192) upon application of a stimulus (18, 28, 58, 68a, 78, 98a, 108a, 118a, 178, 1008),
wherein, when exposed, the pointed tip (14, 24, 44, 54, 64, 74, 84, 94, 104, 114, 164, 174, 194) is configured to enable the implantable device (10, 20, 40, 50, 60, 70, 80, 90, 100, 110, 130, 150, 160, 170, 190) to be moved through tissue upon application of an external force to the implantable device (10, 20, 40, 50, 60, 70, 80, 90, 100, 110, 130, 150, 160, 170, 190).

2. The implantable device (20, 40, 50, 60, 70, 80, 90, 100, 110, 170) as claimed in claim 1, wherein the pointed tip (24, 44, 54, 64, 74, 84, 94, 104, 114, 174) is housed within the body (22, 42, 52, 62, 72, 82, 92, 102, 112, 172) and the part (26, 46, 56a, 66a, 66b, 66c, 76, 86, 96a, 96b, 106a, 106b, 116a, 116b, 116c, 182, 184) is operable to expose the pointed tip (24, 44, 54, 64, 74, 84, 94, 104, 114, 174) by releasing the pointed tip (24, 44, 54, 64, 74, 84, 94, 104, 114, 174) from the end of the body (22, 42, 52, 62, 72, 82, 92, 102, 112, 172).

3. The implantable device (20, 40, 50, 60, 70, 80, 90, 100, 110, 170) as claimed in claim 2, wherein the pointed tip (24, 44, 54, 64, 74, 84, 94, 104, 114, 174) is operable to retract into the end of the body (22, 42, 52, 62, 72, 82, 92, 102, 112, 172) on application of another stimulus.

4. The implantable device (10, 20, 40, 50, 60, 70, 80, 90, 100, 110, 130, 150, 160, 170, 190) as claimed in any one of the preceding claims, wherein the stimulus (18, 28, 58, 68a, 78, 98a, 108a, 118a, 178, 1008) comprises anyone or more of:
an acoustic stimulus
a chemical stimulus;
a mechanical stimulus;
a magnetic stimulus;
a thermal stimulus;
an electric stimulus; and
an electromagnetic radiation stimulus.

5. The implantable device (10, 160, 190) as claimed in any one of claims 1 to 4, wherein the part (16, 166, 196) operable to expose the pointed tip (14, 164, 194) comprises a dissolvable cap (16, 166, 196) covering the pointed tip (14, 164, 194) and the stimulus (18, 1008) comprises a chemical that dissolves the cap (16, 166, 196) to expose the pointed tip (14, 164, 194) at the end of the body (12, 162, 192) when the chemical (18, 1008) is administered.

6. The implantable device as claimed in any one of claims 1 to 4, wherein the part operable to expose the pointed tip comprises a meltable cap covering the pointed tip and the stimulus induces heat in the cap that melts the cap to expose the pointed tip at the end of the body when the stimulus is applied.

7. The implantable device (20) as claimed in any one of claims 1 to 4, wherein the part (20) operable to expose the pointed tip (24) comprises a dissolvable structure (26a) holding the pointed tip (24) within the body (22) and the stimulus (28) comprises a chemical that dissolves the structure (26a) to release the pointed tip (24) from the end of the body (22) when the chemical is administered.

8. The implantable device (40) as claimed in any one of claims 1 to 4, wherein the part (46) operable to expose the pointed tip (44) comprises a spring (46) held in a compressed state by a mechanism (48) and the stimulus comprises a disengagement of the mechanism (48) to decompress the spring (46) to release the pointed tip (44) from the end of the body (42).

9. The implantable device (50) as claimed in any one of claims 1 to 4, wherein the part operable to expose the pointed tip (54) comprises a compressible portion (56b) and a rigid portion (56c) and the stimulus comprises a force (58) that compresses the compressible portion (56b) against the rigid portion (56c) to release the pointed tip (54) from the end of the body (52).

10. The implantable device as claimed in any one of claims 1 to 4, wherein the part operable to expose the pointed tip comprises a cap covering the pointed tip and the stimulus comprises a force that breaks the cap to expose the pointed tip at the end of the body when the force is applied to the cap.

11. The implantable device (60) as claimed in any one of claims 1 to 4, wherein the part (66a, 66b, 66c) operable to expose the pointed tip (64) comprises a flexible portion (66a) and the stimulus comprises a force (68a) that bends the flexible portion (66a) to release the pointed tip (64) from the end of the body (62) when the force (68a) is applied to the flexible portion (66a).

12. The implantable device as claimed in any one of claims 1 to 4, wherein the part operable to expose the pointed tip comprises an expandable body and the stimulus comprises a catalyst that causes expansion of the expandable body to release the pointed tip from the end of the body when the expandable body is exposed to the catalyst.

13. The implantable device as claimed in any one of claims 1 to 4, wherein the part operable to expose the pointed tip comprises a mechanism tuned to disengage at a predefined frequency and the stimulus comprises a vibration at the predefined frequency to disengage the mechanism to release the pointed tip from the end of the body when the vibration is applied.

14. The implantable device (70) as claimed in any one of claims 1 to 4, wherein the part (76) operable to expose the pointed tip (74) and the pointed tip (74) are threadably engaged and the stimulus comprises a rotating magnetic field (78) that rotates the pointed tip (74) along the part (76) to release the pointed tip (74) from the end of the body (72) when the rotating magnetic field (78) is applied.

15. The implantable device (90, 100, 110) as claimed in any one of claims 1 to 4, wherein the part operable to expose the pointed tip (94, 104, 114) comprises a component (96a, 96b, 106a, 106b, 116a, 116b, 116c) configured for activation by a magnetic field (108a), an electromagnetic field (98a), or a radiofrequency signal (118a) and the stimulus comprises the magnetic field (108a), electromagnetic field (98a), or radiofrequency signal (118a) that activates the component (96a, 96b, 106a, 106b, 116a, 116b, 116c) to release the pointed tip (94, 104, 114) from the end of the body (92, 102, 112) when the magnetic field (108a), electromagnetic field (98a) or radiofrequency signal (118a) is applied.
